# EUROPÄISCHE PATENTSCHRIFT

(11) **EP 1 362 045 B1**
(45) Veröffentlichungstag und Bekanntmachung des Hinweises auf die Patenterteilung: **17.11.2004**
(21) Anmeldenummer: 02716791.5
(22) Anmeldetag: 19.02.2002
(51) Int. Cl.: C07D 401/04, A61K 31/4164, A61P 43/00, C07D 401/14

(54) **2-THIO-SUBSITUIERTE IMIDAZOLDERIVATIVE UND IHRE VERWENDUNG IN DER PHARMAZIE**
2-THIO-SUBSTITUTED IMIDAZOLE DERIVATIVES AND THE USE THEREOF IN THE PHARMACEUTICAL INDUSTRY
DERIVES D'IMIDAZOLE SUBSTITUES PAR THIO EN POSITION 2 ET LEUR UTILISATION EN PHARMACIE

(30) Priorität: 19.02.2001 DE 10107683
(43) Veröffentlichungstag der Anmeldung: 19.11.2003
(73) Patentinhaber: MERCKLE GMBH, D-89079 Ulm (DE)
(72) Erfinder: LAUFER, Stefan, 89143 Blaubeuren (DE); KOTSCHENREUTHER, Dunja, 89079 Ulm (DE); MERCKLE, Philipp, 89143 Blaubeuren-Weiler (DE); TOLLMANN, Karola, 65110 Brechen (DE); STRIEGEL, Hans-Günter, 89134 Blaustein (DE)
(74) Vertreter: Riedl, Peter, Dr.
(86) Internationale Anmeldenummer: PCT/EP2002/001746
(87) Internationale Veröffentlichungsnummer: WO 2002/066458

(56) Entgegenhaltungen:
- EP-A- 0 004 648
- WO-A-93/14081
- WO-A-99/03837
- DE-A- 3 504 678
- GB-A- 1 155 580
- US-A- 4 402 960
- GRZEGORZ MLOSTON ET AL.: "Trapping of a thiocarbonyl ylide with imidazolethiones, pyrimidinethione, and thioamides" HELVETICA CHIMICA ACTA., Bd. 82, Nr. 2, - 1990 Seiten 290-296, XP002209741 VERLAG HELVETICA CHIMICA ACTA. BASEL., CH ISSN: 0018-019X
- GRZEGORZ MLOSTON ET AL.: "First examples of reactions of azole N-oxides with thioketones: a novel type of sulfur-transfer reaction" HELVETICA CHIMICA ACTA., Bd. 81, Nr. 9, - 1998 Seiten 1585-1595, XP002209742 VERLAG HELVETICA CHIMICA ACTA. BASEL., CH ISSN: 0018-019X
- CHEMICAL ABSTRACTS, vol. 117, no. 17, 26. Oktober 1992 (1992-10-26) Columbus, Ohio, US; abstract no. 166037b, BEACH, DOROTHY C. ET AL.: "Inhibition of peroxidation by iron(III) and ascorbate." XP002209743 -& DATABASE CAPLUS [Online] CHEMICAL ABSTRACTS SERVICE, COLUMBUS, OHIO, US; CA 117:166037, XP002209744 & ARCH. BIOCHEM. BIOPHYS., Bd. 297, Nr. 2, - 1992 Seiten 258-264,

## Beschreibung

Die vorliegende Erfindung betrifft 2-Thio-substituierte Imidazolderivate mit immunmodulierender und die Cytokinfreisetzung hemmender Wirkung, pharmazeutische Mittel, welche die Verbindungen enthalten, und ihre Verwendung in der Pharmazie.

Pharmakologisch wirksame Imidazolverbindungen, die eine antiinflammatorische Aktivität besitzen, sind bereits bekannt.
So wurden unter anderem Verbindungen mit 4,5-Di(hetero)arylimidazol-Elementen näher untersucht und verschiedene pharmazeutische Wirkungen davon beschrieben. Es sind auch Verbindungen bekannt, die an der 2-Position eine Substitution aufweisen. Das US-Patent 4,585,771 offenbart 4,5-Diphenylimidazolderivate, die an der 2-Position mit einem Pyrrolyl-, Indolyl-, Imidazolyl- oder Thiazolylrest substituiert sind und eine antiinflammatorische und antiallergische Aktivität besitzen.
Die US-Patente 4,528,298 und 4,402,960 beschreiben 4,5-Di(hetero)arylimidazolderivate, die an der 2-Position über eine Thio-, Sulfinyl- oder Sulfonylgruppe mit einem Phenyl-, Pyridyl-, N-Oxypyridyl-, Pyrimidyl-, Thiazolyl- oder Thienylrest substituiert sind, und eine antiinflammatorische und antiallergische Aktivität besitzen. Die US-Patente 4,461,770 und 4,584,310 beschreiben 4-(5-Aryl)-5-(4-heteroaryl)-imidazolderivate, die an der 2-Position über eine Thio-, Sulfinyl- oder Sulfonylgruppe mit einem substituierten oder nicht-substituierten aliphatischen Kohlenwasserstoff substituiert sind, und unter anderem eine antiinflammatorische Wirkung haben.

DE 198 42 833 betrifft 4-Heteroaryl-5-phenylimidazolderivate, die an der 2-Position mit einer Phenylalkylthiogruppe substituiert sind. Diese Verbindungen wirken als Entzündungshemmstoffe und Hemmstoffe der Cytokinfreisetzung. Die WO 99/03837 und WO 93/14081 beschreiben 2-substituierte Imidazole, die die Synthese einer Anzahl inflammatorischer Cytokine hemmen. Die in der WO 93/14081 beschriebenen Verbindungen weisen in der 2-Position einen über ein Schwefelatom gebundenen, phosphorhaltigen Substituenten oder einen Aryl- oder Heteroarylsubstituenten auf. Die WO 91/10662 beschreibt Imidazolderivate, welche die Acyl-Coenzyme A: Cholesterol-O-acyltransferase und die Bindung von Thromboxan TxA₂ inhibieren. Die WO 95/00501 beschreibt Imidazolderivate, die als Cyclooxygenaseinhibitoren brauchbar sind. Die in der DE 28 23 197 A beschriebenen Imidazolderivate besitzen antiinflammatorische, antiallergische und immunstimulierende Wirkung.

J Med. Chem. 1996, 39, 3927-37 beschreibt Verbindungen mit 5-Lipoxygenase- und Cyclooxygenase-inhibierender Wirkung, wobei 2-(4-Methylsulfinylphenyl)-4-(4-fluorphenyl-5-(pyrid-4-yl)-imidazol auch eine Cytokin-inhibierende Wirkung besitzt.

Es wurde gefunden, dass die bekannten Verbindungen nicht stabil und schwer zu verarbeiten sind oder eine geringe Wirksamkeit aufweisen.

Trotz der zahlreichen bekannten Verbindungen besteht daher weiterhin ein Bedarf nach Verbindungen mit antiinflammatorischer Wirkung, die die Cytokinfreisetzung hemmen.

Die Aufgabe der Erfindung ist die Bereitstellung solcher Verbindungen.

Überraschenderweise wurde nun gefunden, dass bestimmte 2-substituierte Imidazolderivate stabile, leicht zu verarbeitende Verbindungen bereitstellen, die eine hohe immunmodulierende und/oder die Cytokinfreisetzung hemmende Wirksamkeit aufweisen.

Gegenstand der vorliegenden Erfindung sind daher die 2-Thio-substituierten Imidazolderivate der Formel I 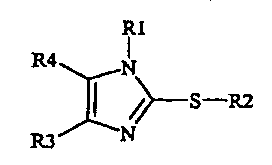 worin
R¹ ausgewählt ist unter:
C₁-C₆-Alkyl, das gegebenenfalls durch eine oder zwei Hydroxy- oder C₁-C₄-Alkoxygruppen, oder einen nicht-aromatischen heterocyclischen Rest mit 5 oder 6 Ringatomen und 1 oder 2 Heteroatomen, die unabhängig voneinander ausgewählt sind unter N, O und S, substituiert ist,
C₂-C₆-Alkenyl,
C₃-C₆-Cycloalkyl,
Aryl, das gegebenenfalls durch ein oder mehrere Halogenatome oder eine C₁-C₄-Alkylsulfanylgruppe substituiert ist,
Amino-C₁-C₄-alkyl, wobei die Aminogruppe gegebenenfalls durch ein oder zwei C₁-C₄-Alkylgruppen substituiert ist,
Aminoaryl, wobei die Aminogruppe gegebenenfalls durch ein oder zwei C₁-C₄-Alkylgruppen substituiert ist,
Aryl-C₁-C₄₋alkyl oder
einem aromatischen oder nicht-aromatischen heterocyclischen Rest mit 5 oder 6 Ringatomen und 1 oder 2 Heteroatomen, die unabhängig voneinander ausgewählt sind unter N, O und S, der gegebenenfalls durch 1, 2, 3 oder 4 C₁-C₄-Alkylgruppen, eine Aryl- oder Aryl-C₁-C₄-alkylgruppe substituiert ist,
R² ausgewählt ist unter:
H;
C₁-C₆-Alkyl,
Phenyl-C₁-C₄-alkyl, wobei die Phenylgruppe einen oder zwei Substituenten aufweisen kann, die unabhängig voneinander unter C₁-C₄-Alkyl, Halogen, C₁-C₄-Alkylsulfanyl, C₁-C₄-Alkylsulfinyl und C₁-C₄-Alkylsulfonyl ausgewählt sind,
C₂-C₆-Alkenyl,
C₂-C₆-Alkenyl, das durch ein oder zwei Halogenatome und/oder Phenylgruppen substituiert ist, wobei die Phenylgruppe unabhängig durch ein oder zwei C₁-C₄-Alkyl oder Halogenatome substituiert sein kann,
C₂-C₆-Alkinyl,
C₂-C₆-Alkinyl, das durch eine Phenylgruppe substituiert ist, die gegebenenfalls durch ein oder zwei C₁-C₄-Alkyl oder Halogenatome substituiert sein kann,
C₁-C₆-Alkyl, das durch einen nicht-aromatischen heterocyclischen Rest mit 5 oder 6 Ringatomen und 1 oder 2 Heteroatomen, die unabhängig voneinander ausgewählt sind unter N, O, und S, C₁-C₄-Alkylsulfanyl, C₁-C₄-Alkylsulfinyl oder C₁-C₄-Alkylsulfonyl substituiert ist,
Phenyl oder
Phenyl steht, das einen oder zwei Substituenten aufweist, die unabhängig voneinander unter C₁-C₄-Alkyl, Halogen, C₁-C₄-Alkylsulfanyl, C₁-C₄-Alkytsulfinyl oder C₁-C₄-Alkylsulfonyl ausgewählt sind, oder
R¹ und R² zusammen für -CH₂CH₂-oder -CH₂CH₂CH₂- stehen,
   einer der Reste R³ und R⁴ für C₁-C₆-Alkyl oder einen aromatischen heterocyclischen Rest mit 5 oder 6 Ringatomen und 1 oder 2 Heteroatomen, die unabhängig voneinander ausgewählt sind unter N, O und S, steht, wobei der aromatische heterocyclische Rest 1 oder 2 Substituenten aufweisen kann, die unabhängig voneinander ausgewählt sind unter C₁-C₆-Alkyl, Amino, C₁-C₄-Alkylamino, Di-C₁-C₄-alkylamino, Phenyl-C₁-C₄-alkylamino und R⁵CONR⁶-, worin R⁵ für C₁-C₄-Alkyl, Phenyl, das einen oder zwei Substituenten aufweisen kann, die unabhängig voneinander unter C₁-C₄-Alkyl, C₁-C₄-Alkoxy und Halogen ausgewählt sind, oder C₃-C₆-Cycloalkyl steht und R⁶ für H, C₁-C₄-Alkyl oder Benzyl steht, und
   der zweite der Reste R³ und R⁴ für C₁-C₆-Alkyl oder Aryl steht, das gegebenenfalls durch ein Halogenatom substituiert ist, wobei nur einer der Reste R³ und R⁴ für C₁-C₆-Alkyl stehen kann,
   mit der Einschränkung, dass wenn R¹ für Aryl-C₁-C₅-alkyl oder Amino-C₁-C₆-alkyl steht, R² für Alkylsulfonyl- oder Alkylsulfinyl-aryl-C₁-C₅-alkyl steht,
und die optischen Isomere und physiologisch verträglichen Salze davon.

Bevorzugt sind Verbindungen der Formel I, worin R¹ für C₁-C₆-Alkyl, das gegebenenfalls durch eine oder zwei Hydroxygruppen oder einen nicht-aromatischen heterocyclischen Rest substituiert ist, C₂-C₆-Alkenyl, C₃-C₆-Cycloalkyl, Aryl, das gegebenenfalls durch ein oder mehrere Halogenatome oder eine C₁-C₄-Alkylsulfanylgruppe substituiert ist, Amino-C₁-C₄-alkyl, wobei die Aminogruppe gegebenenfalls durch ein oder zwei C₁-C₄-Alkylgruppen substituiert ist, Aminoaryl, wobei die Aminogruppe gegebenenfalls durch ein oder zwei C₁-C₄-Alkylgruppen substituiert ist, Aryl-C₁-C₄-alkyl oder einen aromatischen oder nicht-aromatischen heterocyclischen Rest mit 5 oder 6 Ringatomen und 1 oder 2 Heteroatomen, die unabhängig voneinander ausgewählt sind unter N, O und S, der gegebenenfalls durch 1, 2, 3 oder 4 C₁-C₄-Alkylgruppen, eine Aryl- oder Aryl-C₁-C₄-alkylgruppe substituiert ist, steht,
R² für H, C₁-C₆-Alkyl, Phenyl-C₁-C₄-alkyl, wobei die Phenylgruppe einen oder zwei Substituenten aufweisen kann, die unabhängig voneinander unter C₁-C₄-Alkyl; Halogen, C₁-C₄-Alkylsulfanyl, C₁-C₄-Alkylsulfinyl und C₁-C₄-Alkylsulfonyl ausgewählt sind, C₂-C₆-Alkenyl, C₂-C₆-Alkenyl, das durch ein oder zwei Halogenatome und/oder Phenylgruppen substituiert ist, wobei die Phenylgruppe unabhängig durch ein oder zwei C₁-C₄-Alkyl oder Halogenatome substituiert sein kann, C₂-C₆-Alkinyl, C₂-C₆-Alkinyl, das durch eine Phenylgruppe substituiert ist, die gegebenenfalls durch ein oder zwei C₁-C₄-Alkyl oder Halogenatome substituiert sein kann, C₁-C₆-Alkyl, das durch einen nicht-aromatischen heterocyclischen Rest mit 5 oder 6 Ringatomen und 1 oder 2 Heteroatomen, die unabhängig voneinander ausgewählt sind unter N, O, und S, substituiert ist; steht,
einer der Reste R³ und R⁴ für C₁-C₆-Alkyl oder einen aromatischen heterocyclischen Rest mit 5 oder 6 Ringatomen und 1 oder 2 Heteroatomen, die unabhängig voneinander ausgewählt sind unter N, O und S, steht, wobei der aromatische heterocyclische Rest 1 oder 2 Substituenten aufweisen kann, die unabhängig voneinander ausgewählt sind unter C₁-C₆-Alkyl, Amino, C₁-C₄-Alkylamino, Di-C₁-C₄-alkylamino, Phenyl-C₁-C₄-alkylamino, R⁵CONR⁶-, worin R⁵ für C₁-C₄-Alkyl, Phenyl, das einen oder zwei Substituenten aufweisen kann, die unabhängig voneinander unter C₁-C₄-Alkyl, C₁-C₄-Alkoxy und Halogen ausgewählt sind, oder C₃-C₆-Cycloalkyl steht und R⁶ für H, C₁-C₄-Alkyl oder Benzyl steht, und der zweite der Reste R³ und R⁴ für C₁-C₆-Alkyl oder Aryl steht, das gegebenenfalls durch ein Halogenatom substituiert ist, wobei nur einer der Reste R³ und R⁴ für C₁-C₆-Alkyl stehen kann.

Soweit die erfindungsgemäßen Verbindungen Asymetriezentren aufweisen, sind Racemate sowie optische Isomere (Enantiomere, Diastereomere) umfasst.

Der Ausdruck "Alkyl" (auch in Verbindungen mit anderen Gruppen, wie Phenylalkyl, Alkylsulfonyl etc.) umfasst geradkettige und verzweigte Alkylgruppen mit vorzugsweise 1 bis 6 bzw. 1 bis 4 C-Atomen, wie Methyl, Ethyl, n- und i-Propyl, n-, i- und t-Butyl, sec-Butyl, n-Pentyl und n-Hexyl.

Der Ausdruck "Aryl" umfasst aromatische Ringsysteme wie Phenyl oder Naphthyl.

Der Ausdruck "Halogen" steht für ein Fluor-, Chlor-, Brom- oder lodatom, insbesondere ein Fluor - oder Chloratom.

C₃-C₆-Cycloalkylgruppen sind Cyclopropyl, Cyclobutyl und insbesondere Cyclopentyl und Cyclohexyl.

Nicht-aromatische heterocyclische Reste können gesättigt oder ungesättigt sein. Bevorzugt sind Piperidinyl, Piperazinyl, Pyranyl, Morpholinyl oder Pyrrolidinyl, wobei der Piperidinylrest durch 1, 2, 3 oder 4 C₁-C₄-Alkylgruppen, insbesondere Methylgruppen; substituiert sein kann.

Bevorzugte aromatische heterocyclische Reste sind Pyridyl, insbesondere 3- oder 4-Pyridyl, Pyrimidinyl, Pyrrolyl, Imidazolyl, Oxazolyl, Isoxazolyl, Furyl, Thienyl oder Thiazolyl. Der heterocyclische Rest, insbesondere der Pyridylrest, kann wie oben angegeben substituiert sein. Der Pyridylrest ist insbesondere in 2-Position substituiert.

Phenyl-C₁-C₄-alkyl bedeutet insbesondere Benzyl oder Phenylethyl.

Bevorzugt sind Verbindungen der Formel I, wobei einer der Reste R³ und R⁴ für C₁-C₄-Alkyl oder ein halogensubstituiertes Phenyl steht und der zweite der Reste R³ und R⁴ für C₁-C₄-Alkyl oder Pyridyl oder substituiertes Pyridyl steht, jedoch nicht beide Reste für C₁-C₄-Alkyl stehen.

Weiterhin bevorzugt sind Verbindungen der Formel I, wobei R³ für halogensubstituiertes, insbesondere 4-substituiertes, Phenyl und R⁴ für gegebenenfalls substituiertes Pyridyl, insbesondere 4-Pyridyl oder substituiertes 4-Pyridyl, steht.

Gemäß einer besonders bevorzugten Ausführungsform steht der Rest R³ in der Formel I für 4-Fluorphenyl und R⁴ für 4-Pyridyl oder substituiertes Pyridyl.

Wenn R¹ für C₁-C₆-Alkyl steht, das durch einen nicht-aromatischen heterocyclischen Rest substituiert ist, enthält dieser vorzugsweise mindestens ein Stickstoffatom, wobei die Bindung an die Alkylgruppe bevorzugt über das Stickstoffatom erfolgt.

Wenn R¹ für einen aromatischen oder nicht-aromatischen heterocyclischen Rest steht, ist dieser bevorzugt über ein Kohlenstoffatom an die Imidazolgruppe gebunden.

R¹ steht vorzugsweise für C₁-C₃-Alkyl, C₃-C₆-Cycloalkyl, insbesondere Cyclopropyl, oder einen gesättigten heterocyclischen Rest mit einem oder zwei N-Atomen, insbesondere Piperidinyl oder 2,2,6,6-Tetramethylpiperidinyl. Besonders bevorzugt ist der Piperidinyl- oder 2,2,6,6-Tetramethylpiperidinylrest in 4-Position an das Stickstoffatom des Imidazols gebunden.

R² steht vorzugsweise für C₁-C₃-Alkyl (Methyl, Ethyl, n-Propyl oder i-Propyl), Phenyl-C₁-C₄-alkyl, insbesondere Benzyl, das gegebenenfalls wie oben angegeben substituiert ist. Besonders bevorzugt steht R² für C₁-C₃-Alkyl oder Benzyl, das durch C₁-C₄-Alkyl-sulfanyl, C₁-C₄-Alkylsulfinyl oder C₁-C₄-Alkylsulfonyl substituiert ist, insbesondere in 4-Position.

Besonders bevorzugt sind Verbindungen der Formel I, worin R⁴ für Pyridyl, insbesondere 4-Pyridyl, das durch Amino, C₁-C₄-Alkylamino oder R⁵COR⁶-, worin R⁵ und R⁶ die oben angegebenen Bedeutungen besitzen, substituiert ist, R¹ für C₁-C₃-Alkyl und R² für C₁-C₃-Alkyl stehen.

Die physiologisch verträglichen Salze können im vorliegenden Fall Säureadditions- oder Basenadditionssalze sein. Für Säureadditionssalze werden anorganische Säuren wie Salzsäure, Schwefelsäure oder Phosphorsäure eingesetzt oder organische Säuren wie Weinsäure, Citronensäure, Maleinsäure, Fumarsäure, Äpfelsäure, Mandelsäure, Ascorbinsäure, Gluconsäure und dergleichen.

Die Herstellung der erfindungsgemäßen Verbindungen mit R²≠ H erfolgt in einem zweistufigen Verfahren. Im ersten Schritt wird zunächst ein substituiertes Imidazol-2-thion (R² = H) hergestellt. Dieses wird dann im zweiten Schritt so umgesetzt, dass der gewünschte Substituent eingeführt wird.

### 1) Herstellung des Imidazol-2-thions

Zur Herstellung des Imidazol-2-thions stehen zwei Verfahrensvarianten zur Verfügung. Die beiden Varianten werden beispielhaft an Verbindungen erläutert, worin R³ für 4-Fluorphenyl und R⁴ für 4-Pyridyl steht. Verbindungen mit anderen Resten R³ und R⁴ können in entsprechender Weise hergestellt werden.

### Variante 1

Die Synthese der substituierten Imidazol-2-thione erfolgt ausgehend von Isonicotinsäureethylester und 4-Fluorphenylacetonitril nach dem Reaktionsablauf nach Schema 1.

Die Ausgangsstoffe werden in einer Kondensationsreaktion mit Hilfe von metallischen Natrium in einem Alkohol, z.B. Ethanol, zu 2-Cyano-2-(4-fluorphenyl)-1-(4-pyridyl)-ethanon (Verbindung 1) umgesetzt. Anschließend wird die Cyanogruppe durch Hydrolyse, z.B. mit Bromwasserstoffsäure, und Decarboxylierung entfernt, so dass 2-(4-Fluorphenyl)-1-(4-pyridyl)-ethanon (Verbindung 2) entsteht. Im nächsten Schritt wird die Verbindung 2 in der 2-Position nitrosiert, z.B. unter Verwendung von Nitriten, wie Natriumnitrit oder Isoamylnitrit. Dabei entsteht die Verbindung der Formel (3), das Oxim 2-(4-Fluorphenyl)-1-(4-pyridyl)-α-hydroxyiminoethan.

Ausgehend von dieser Zwischenverbindung erfolgt durch Umsetzung mit einem Imin der allgemeinen Formel H₂C=NR₁, das als 1,3,5-trisubstituiertes Hexahydro-1,3,5-triazin vorliegt, in einem alkoholischen Lösungsmittel, wie Ethanol, und bei erhöhter Temperatur (50-90°C) der Ringschluss zu einem Imidazolderivat der Formel (4), einem substituierten 5-(4-Fluorphenyl)-4-(4-pyridyl)-imidazol-N-oxid, das am Stickstoffatom in 3-Position den Substituenten R₁ trägt. Das Imidazol-N-oxid der Formel (4) wird dann mit 2,2,4,4-Tetramethyl-3-thio-cyclobutanon in einem chlorierten Lösungsmittel zu dem entsprechenden 3-substituierten 5-(4-Fluorphenyl)-4-(4-pyridyl)-imidazol-2-thion (Verbindung 5; Verbindung der Formel I mit R² = H) umgesetzt.

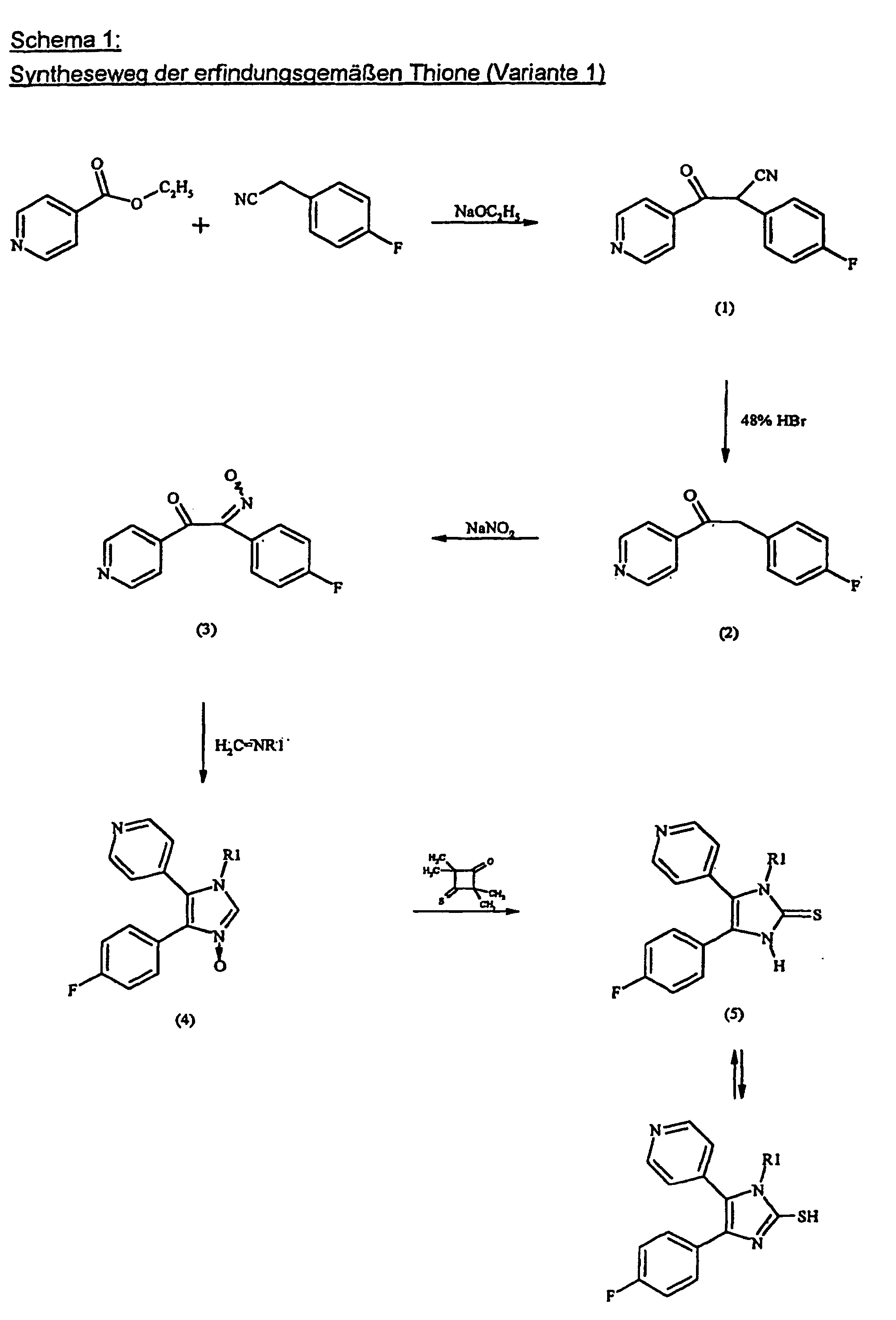

### Variante 2

Zunächst wird die Oximverbindung der Formel (3), 2-(4-Fluorphenyl)-1-(4-pyridyl)-α-hydroxyiminoethan wie in Variante 1 (Schema 1, Schritte 1 bis 3) beschrieben hergestellt. Davon ausgehend erfolgt die Synthese der substituierten Imidazol-2-thione gemäß Schema 2.

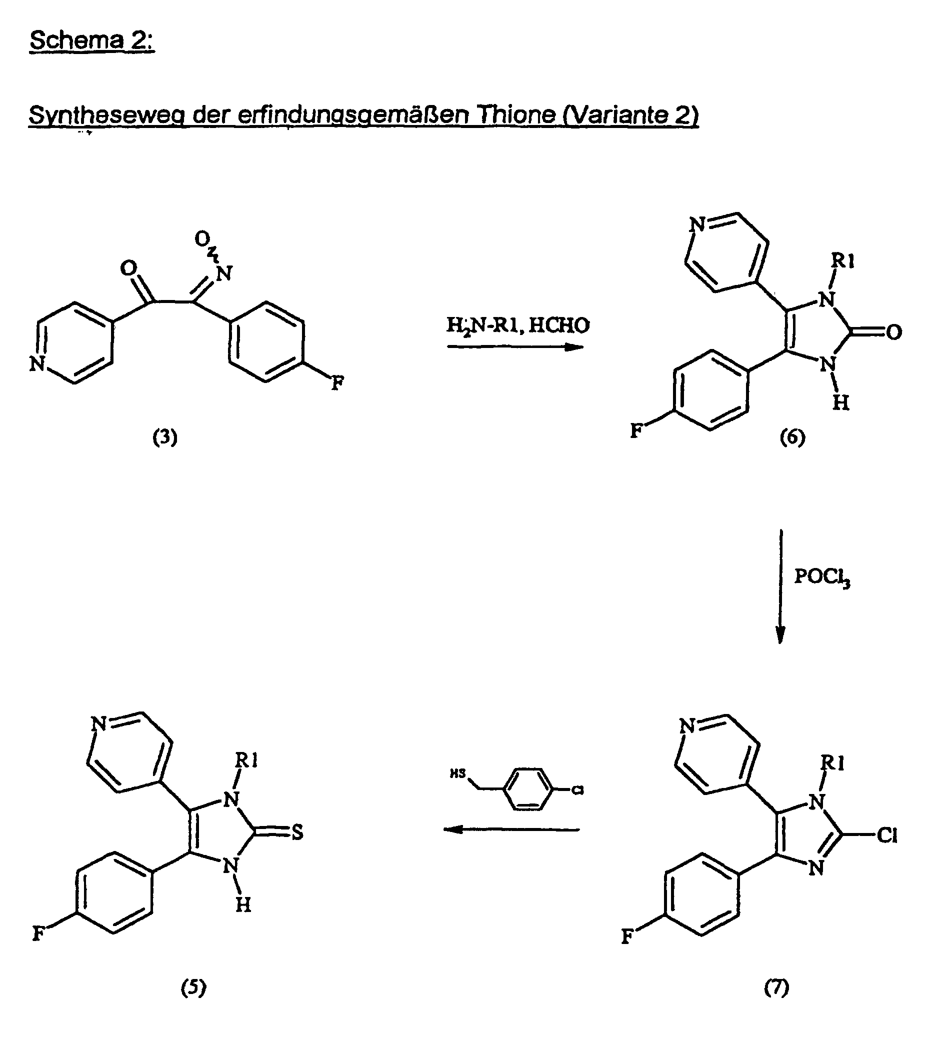

2-(4-Fluorphenyl)-1-(4-pyridyl)-α-hydroxyiminoethan wird gemäß Schema 2 mit dem ausgewählten Amin der allgemeinen Formel NH₂₋R₁ und Formaldehyd umgesetzt, wobei unter Ringschluss eine Verbindung der Formel (6), ein 1-substituiertes 4-(4-Fluorphenyl)-5-(4-pyridyl)-imidazol-2-on, entsteht. Dieses wird mit Phosphoroxychlorid im Überschuss umgesetzt, wobei eine Verbindung der Formel (7), ein 1-substituiertes 4-(4-Fluorphenyl)-5-(4-pyridyl)-imidazol-2-chlorid, gebildet wird. Aus dieser wird das entsprechende 1-substituierte 4-(4-Fluorphenyl)-5-(4-pyridyl)-imidazol-2-thion (Verbindung 5) durch die Umsetzung mit 4-Chlorbenzylthiol in einem polaren aprotischen Lösungsmittel und bei erhöhter Temperatur (100-150°C) gewonnen.

### 2) Herstellung der 2-Thio-imidazolverbindung

Die gemäß Variante 1 oder 2 erhaltenen Thionverbindungen (5) werden durch Substitution des Schwefelatoms in der 2-Position zu den erfindungsgemäßen Verbindungen der Formel I mit R² ≠ H umgesetzt. Die Substitutionen wie beispielhaft für einige Verbindungen in Schema 3 gezeigt erfolgen in bekannter Weise durch eine nukleophile Substitutionsreaktion. Die Verbindung 5 wird dabei mit R²-X in einem inerten polaren Lösungsmittel, wie einem Alkohol, umgesetzt. X steht für eine leicht austauschbare Gruppe, wie Hal, insbesondere Cl, Br, I, Methylsulfonyl, Tosyl etc.

2-Thioimidazolverbindungen, bei denen das S-Atom 2-Position mit einem Vinylrest substituiert ist, können durch nukleophile Addition der Verbindung 5 an eine Dreifachbindung erhalten werden. Zu diesem Zweck wird 5 mit einer Base, z.B. einem Alkalimetallalkoholat in dem entsprechenden Alkohol, und anschließend mit einem Überschuss der Verbindung mit der Dreifachbindung umgesetzt.

Die Herstellung der entsprechenden Bisarylthioether erfolgt ausgehend von dem 3-substituierten 2-Chlor-4-(4-fluorphenyl)-5-(4-pyridyl)-imidazol (Verbindung 7 aus Schema 2). Die Verbindungen (7) werden mit zwei Äquivalenten des entsprechenden Thiophenols in einem aprotischen Lösungsmittel, wie Dimethylformamid, zu Verbindungen der Formel (12) umgesetzt.

Die entsprechenden regioisomeren Verbindungen können gemäß Schema (5) hergestellt werden. Ausgehend von 1-(4-Fluorphenyl)-2-(4-pyridyl)-α-hydroxyiminoethanon (in analoger Weise nach Schema 1 erhalten) werden analog zu dem Verfahren nach Schema 1 durch Umsetzen mit den entsprechenden Iminen Verbindungen der Formel 15 gewonnen. Die Verbindung (13) ist gemäß dem in WO 93/14081 beschriebenen Verfahren herstellbar.

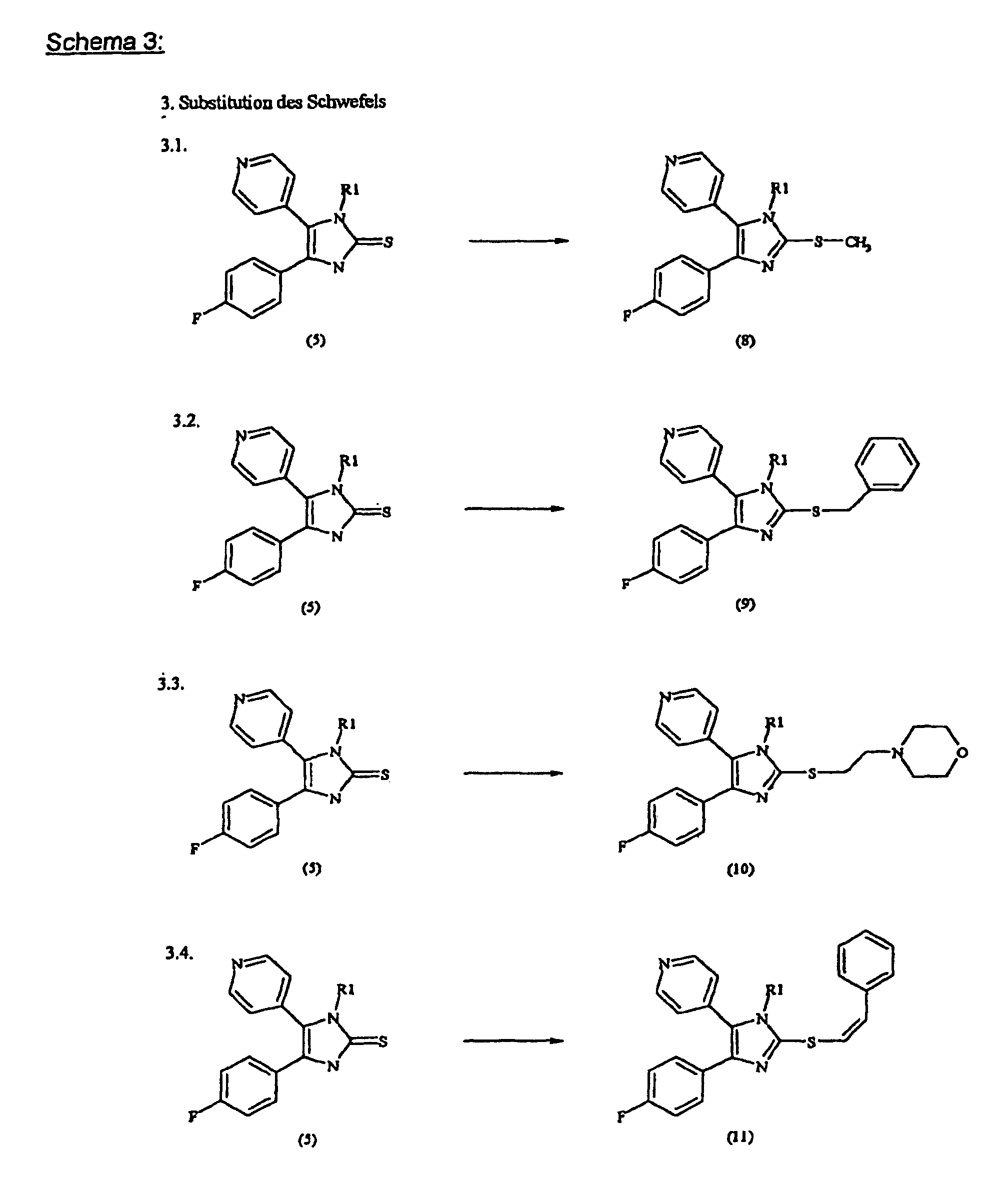

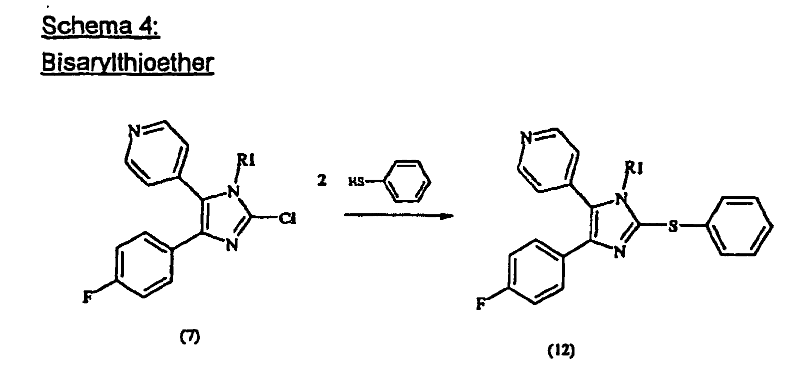

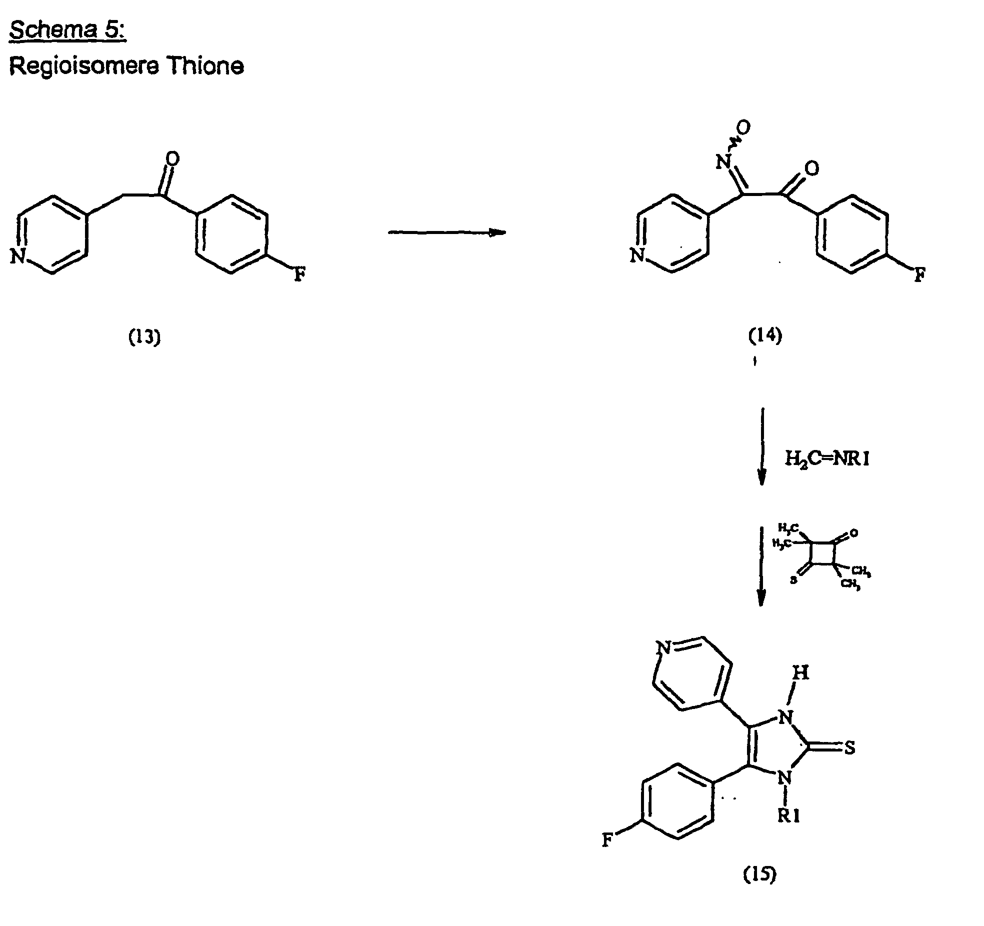

Sie können weiter umgesetzt werden wie in Schema 3 beschrieben.

Die in 4-Position eine C₁-C₄-Alkylgruppe aufweisenden Imidazolthiole werden ausgehend von den entsprechenden α-Hydroxyiminoethanonen (Verbindung 17/19 aus nachstehendem Schema 6) in analoger Weise nach Schema 1 und 2 gewonnen.

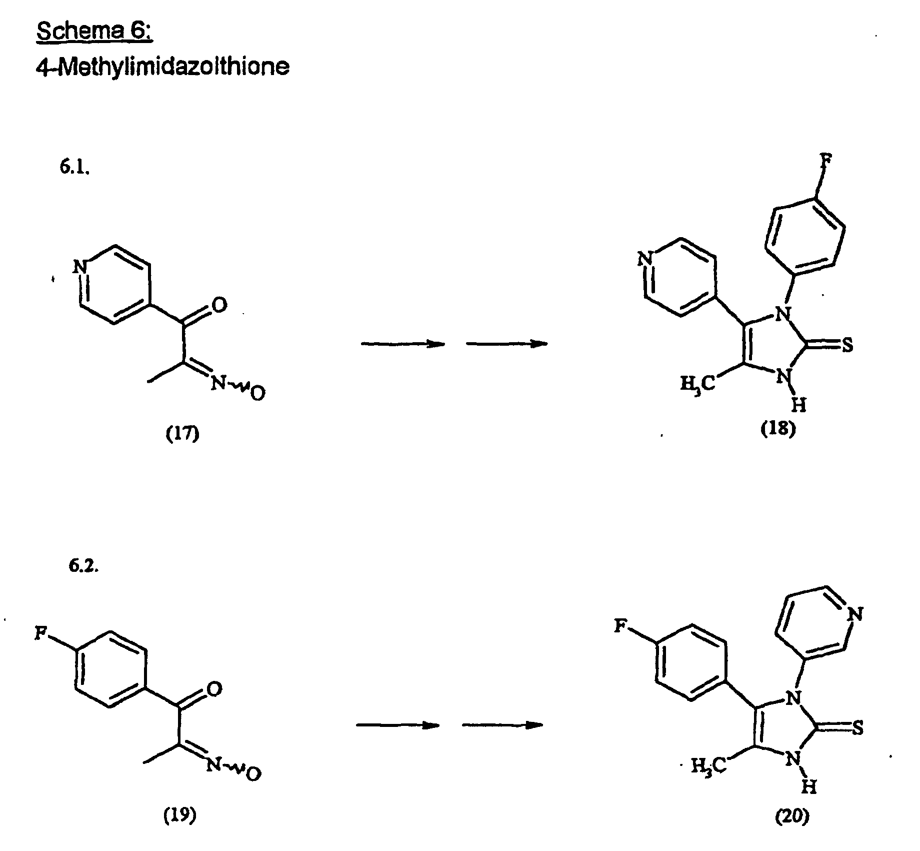

Sie können nach Schema 3 und 4 weiter umgesetzt werden. Die entsprechenden regioisomeren Verbindungen können analog zu Schema 5 hergestellt werden.

Verbindungen der Formel I, die einen C₁-C₄-Alkylsulfanylrest aufweisen, können mit einem geeigneten Oxidationsmittel, wie m-Chlorperbenzoesäure, Wasserstoffperoxid, Benzoylperoxid etc., nach bekannten Verfahren zur entsprechenden C₁-C₄-Alkylsulfinyl- oder C₁-C₄-Alkylsulfonylverbindung oxidiert werden, siehe Schema 7.

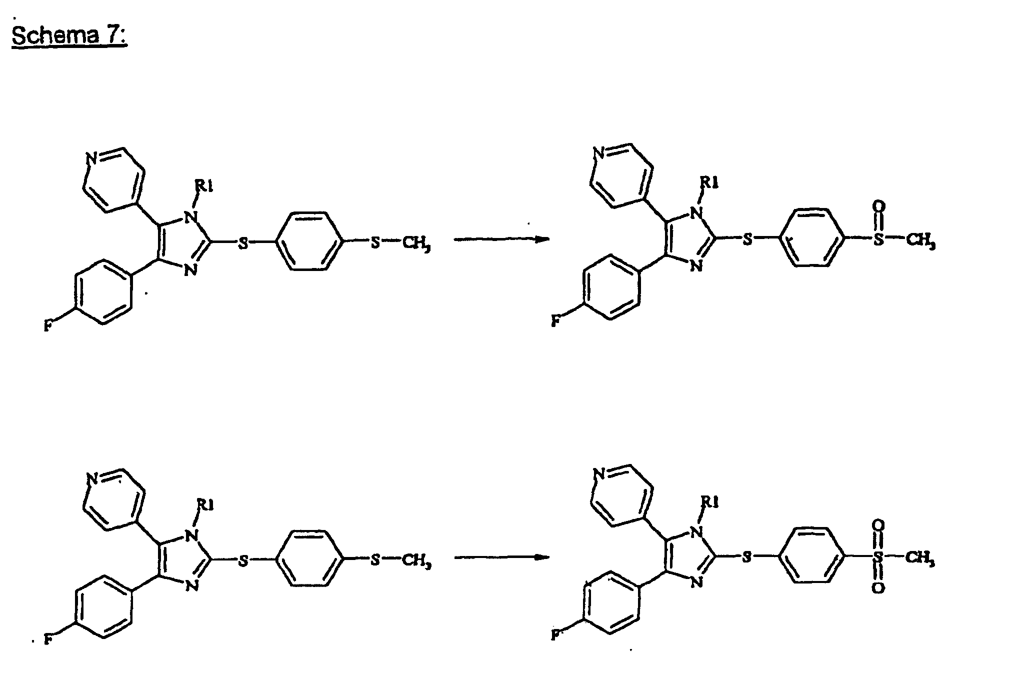

Die Herstellung derjenigen Verbindungen, bei denen R⁴ für einen amino- oder amidosubstituierten heterocyclischen Rest, insbesondere einen Pyridylrest, steht, erfolgt gemäß Schema 8, worin die Herstellung am Beispiel 2-substituierter 4-Pyridinverbindungen erläutert wird (die Herstellung der Verbindungen, bei denen R⁴ für einen alkylsubstituierten heterocyclischen Rest steht, erfolgt nach den oben angegebenen Verfahren mit entsprechend substituierten Ausgangsverbindungen):

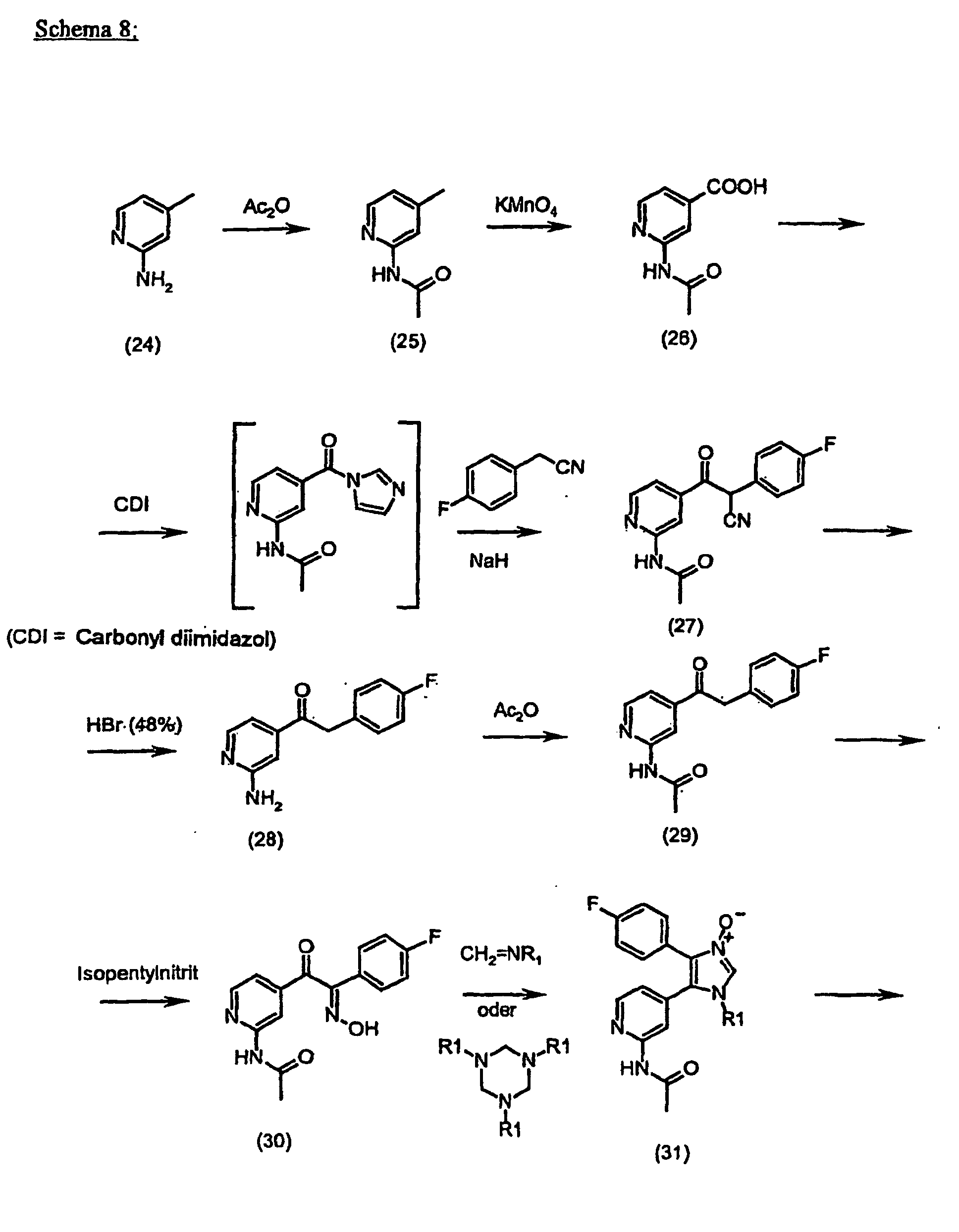

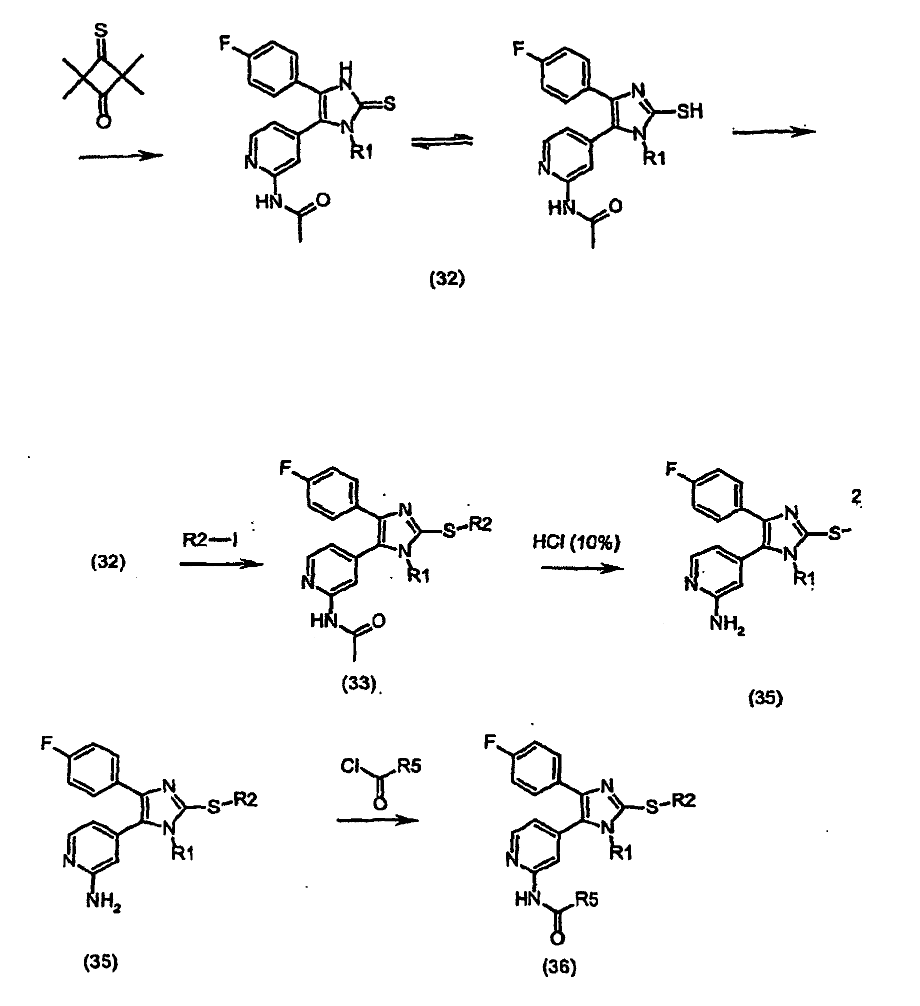

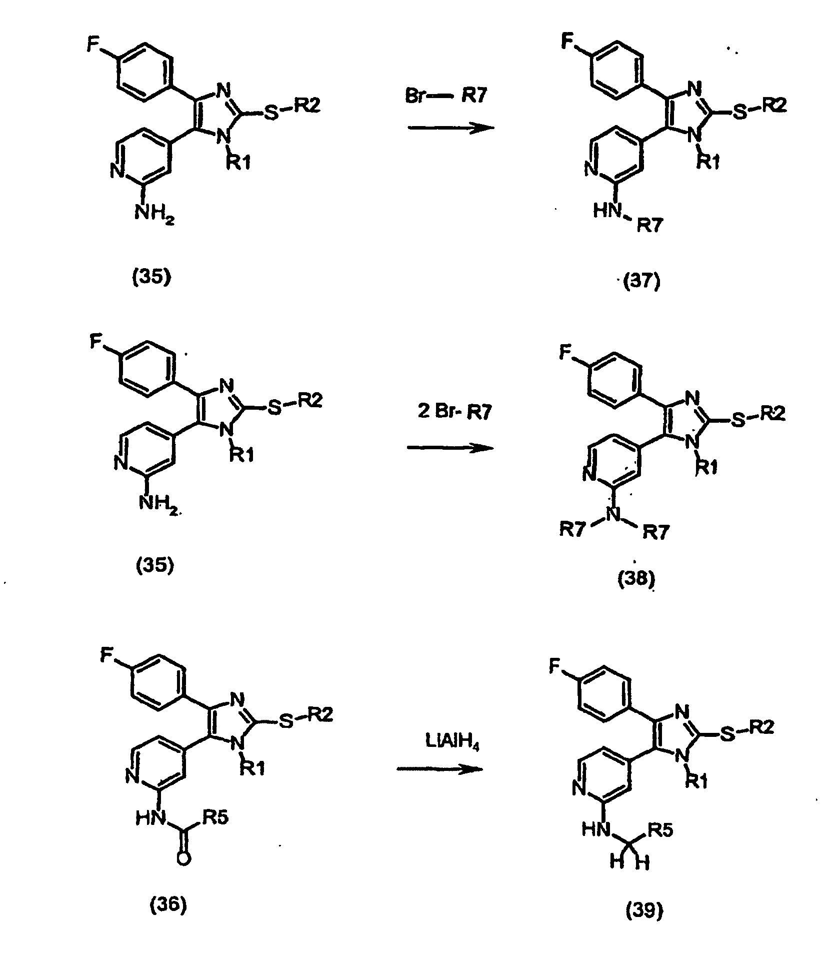

Die Aminogruppe der Ausgangsverbindung 2-Amino-γ-picolin (24) wird geschützt, z.B. durch Einführen einer Acetylgruppe mit Acetanhydrid. Anschließend erfolgt die Oxidation der Methylgruppe der Verbindung (25) zur Carboxylgruppe, z.B. mit Kaliumpermanganat in wässrigem Medium bei 20 bis 90 °C.

Die Umsetzung der erhaltenen Pyridincarbonsäure (26) mit 4-Fluorphenylacetonitril zu Verbindung (27) und die anschließende Abspaltung der Nitrilgruppe werden gemäß Variante 1 durchgeführt. Dabei wird auch die Acetylgruppe an der Aminogruppe der Pyridinverbindung unter Bildung der Verbindung (28) abgespalten.

Im nächsten Schritt wird erneut die Aminogruppe geschützt, z. B. durch Einführen einer Acetylgruppe mit Acetanhydrid. Die erhaltene Verbindung (29) wird gemäß Variante 1 oder 2 (im Schema 8 anhand der Variante 1 gezeigt) in die Thionoverbindung (32) überführt. In diese wird der gewünschte Rest R² eingeführt wie in Schema 3, 4 und 7 beschrieben.

Um den gewünschten Substituenten in die Pyridylgruppe einzuführen, wird zunächst die Acetylgruppe hydrolytisch, z.B. mit wässriger Säure, abgespalten, wobei man die Aminoverbindung (35) erhält. Die Einführung eines Acylrestes erfolgt durch Acylierung, insbesondere mit dem entsprechenden Säurechlorid R⁵COCl in einem inerten Lösungsmittel, wie einem Ether, z.B. Tetrahydrofuran, Dioxan, oder einem chlorierten Kohlenwasserstoff, z.B. Methylenchlorid oder 1,2-Dichlorethan etc. Die Acylierung erfolgt im Allgemeinen in Anwesenheit einer Base, z.B. Triethylamin, in mindestens äquivalenter Menge.

Zur Herstellung der substituierten Aminverbindungen wird Verbindung (35) mit einem oder zwei Moläquivalenten eines Alkylbromids oder Phenylalkyibromids in einem inerten Lösungsmittel, wie Dimethylformamid in Anwesenheit einer Base, wie Natriumhydrid, zu den Verbindungen (37) oder (38) umgesetzt. Alternativ können die Amidverbindungen (34) oder (36) mit Lithiumaluminiumhydrid in z.B. Tetrahydrofuran zu Verbindung 39 reduziert werden.

Die erfindungsgemäßen Verbindungen zeigen *in vitro* und *in vivo* immunmodulierende und die Cytokinfreisetzung hemmende Wirkung. Cytokine sind Proteine wie TNF-α und IL-β, die eine wichtige Rolle bei zahlreichen inflammatorischen Erkrankungen spielen. Die erfindungsgemäßen Verbindungen eignen sich durch ihre die Cytokinfreisetzung hemmende Wirkung zur Behandlung stehen. Sie eignen sich beispielsweise zur Behandlung von Autoimmunerkrankungen, Krebs, rheumatischer Arthritis, Gicht, septischem Schock, Osteoporosis, neuropathischem Schmerz, HIV-Ausbreitung, HIV-Demenz, viraler Myokarditis, insulinabhängiger Diabetis, Periodontalerkrankungen, Restenosis, Alopezie, T-Zell-Depletion bei HIV-Infektionen oder AIDS, Psoriasis, akuter Pankreatitis, Abstoßungsreaktionen bei allogenen Transplantaten, allergisch bedingter Lungenentzündung, Artheriosklerose, Multipler Sklerose, Kachexie, Alzheimer Erkrankung, Schlaganfall, Iktus, Colitis ulcerosa, Morbus Crohn, Inflammatory-Bowel-Disease (IBD), Ischämie, kongestive Herzinsuffizienz, Lungen-Fibrose, Hepatitis, Glioblastom, Guillain-Barre-Syndrom, systematischer Lupus erythematodes, Adult-respiratory-distress-Syndrom (ARDS) und Atemnotsyndrom.

Die erfindungsgemäßen Verbindungen können entweder als einzelne therapeutische Wirkstoffe oder als Mischungen mit anderen therapeutischen Wirkstoffen verabreicht werden. Die Verbindungen können alleine verabreicht werden, im Allgemeinen werden sie jedoch in Form pharmazeutischer Mittel dosiert und verabreicht, d.h. als Mischungen der Wirkstoffe mit geeigneten pharmazeutischen Trägern oder Verdünnungsmittel. Die Verbindungen oder Mittel können oral oder parenteral verabreicht werden, vorzugsweise werden sie in oralen Dosierungsformen gegeben.

Die Art des pharmazeutischen Mittels oder Trägers bzw. des Verdünnungsmittels hängt von der gewünschten Verabreichungsform ab. Orale Mittel können bspw. als Tabletten oder Kapseln vorliegen und können übliche Exzipienzien enthalten wie Bindemittel (z.B. Sirup, Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talcum, Polyethylenglycol oder Siliciumdioxid), desintegrierende Mittel (z. B. Stärke) oder Netzmittel (z. B. Natriumlaurylsulfat). Flüssige Oralpräparate können in Form wässriger oder öliger Suspensionen, Lösungen, Emulsionen, Sirupe, Elixiere oder Sprays und dergleichen sein. Sie können auch als Trockenpulver vorliegen, das zur Rekonstitution mit Wasser oder einem anderen geeigneten Träger aufbereitet wird. Derartige flüssige Präparate können übliche Additive, beispielsweise Suspendiermittel, Geschmacksstoffe, Verdünnungsmittel oder Emulgatoren enthalten. Für die parenterale Verabreichung kann man Lösungen oder Suspensionen mit üblichen pharmazeutischen Trägern einsetzen.

Die erfindungsgemäßen Verbindungen oder Mittel können an Säuger (Mensch oder Tier) in einer Dosis von etwa 0,5 mg bis 100 mg pro kg Körpergewicht pro Tag gegeben werden. Das Wirkungsspektrum der Verbindungen als Inhibitoren der Cytokinfreisetzung wurde anhand nachstehender Testsysteme wie beschrieben von Donat C. und Laufer S. in Arch. Pharm. Pharm. Med. Chem. 333, Suppl. 1, 1-40, 2000 untersucht.

### In-vitro-Testverfahren mit humanem Vollblut

Proben aus humanem Kalium-EDTA-Vollblut (à 400 µl) werden mit der Testsubstanz versetzt und 15 min bei 37°C in einem CO₂-Inkubator (5% CO_{2;} 95% feuchtigkeitsgesättigte Luft) vorinkubiert. Danach werden die Proben 4 Stunden mit 1 µg/ml LPS (*E*.*coli* 026:B6) bei 37°C in einem CO₂-Inkubator (5% CO_{2;} 95% feuchtigkeitsgesättigte Luft) stimuliert. Die Reaktion wird gestoppt, indem die Proben auf Eis gestellt, mit DPBS-Puffer versetzt und anschließend 15 min bei 1000*g zentrifugiert werden. Anschließend wird die Menge IL-1β und TNFα im Plasmaüberstand mittels ELISA ermittelt.

### In-vitro-Testverfahren mit PBMCs

Aus 1:3 verdünntem humanen Kalium-EDTA-Vollblut werden mittels Dichtegradientenzentrifugation (Histopaque®-1,077) die mononukleären Zellen (PBMCs) isoliert, Diese werden 2 mal mit DPBS-Puffer gewaschen, in Makrophagen-SFM- Medium resuspendiert und auf eine Zellzahl von 1*10⁶ Zellen/ml eingestellt.

Die erhaltene PBMCs-Suspension (à 390 µl Proben) wird mit der testsubstanz 15 min bei 37°C in einem CO₂-Inkubator (5% CO_{2;} 95% feuchtigkeitsgesättigte Luft) vorinkubiert. Danach werden die Proben 4 Stunden mit jeweils 1 µl/ml LPS (*E*.*coli* 026:B6) bei 37°C in einem CO₂-Inkubator (5% CO_{2;} 95% feuchtigkeitsgesättigte Luft) stimuliert. Die Reaktion wird gestoppt, indem die Proben auf Eis gestellt, mit DPBS-Puffer versetzt und anschließend 12 min bei 15880*g zentrifugiert werden. Anschließend wird die Menge IL-1β und TNFα im Plasmaüberstand mittels ELISA ermittelt.

Die Ergebnisse der *in-vitro*-Tests sind in den nachstehenden Tabellen 1 und 2 gezeigt.

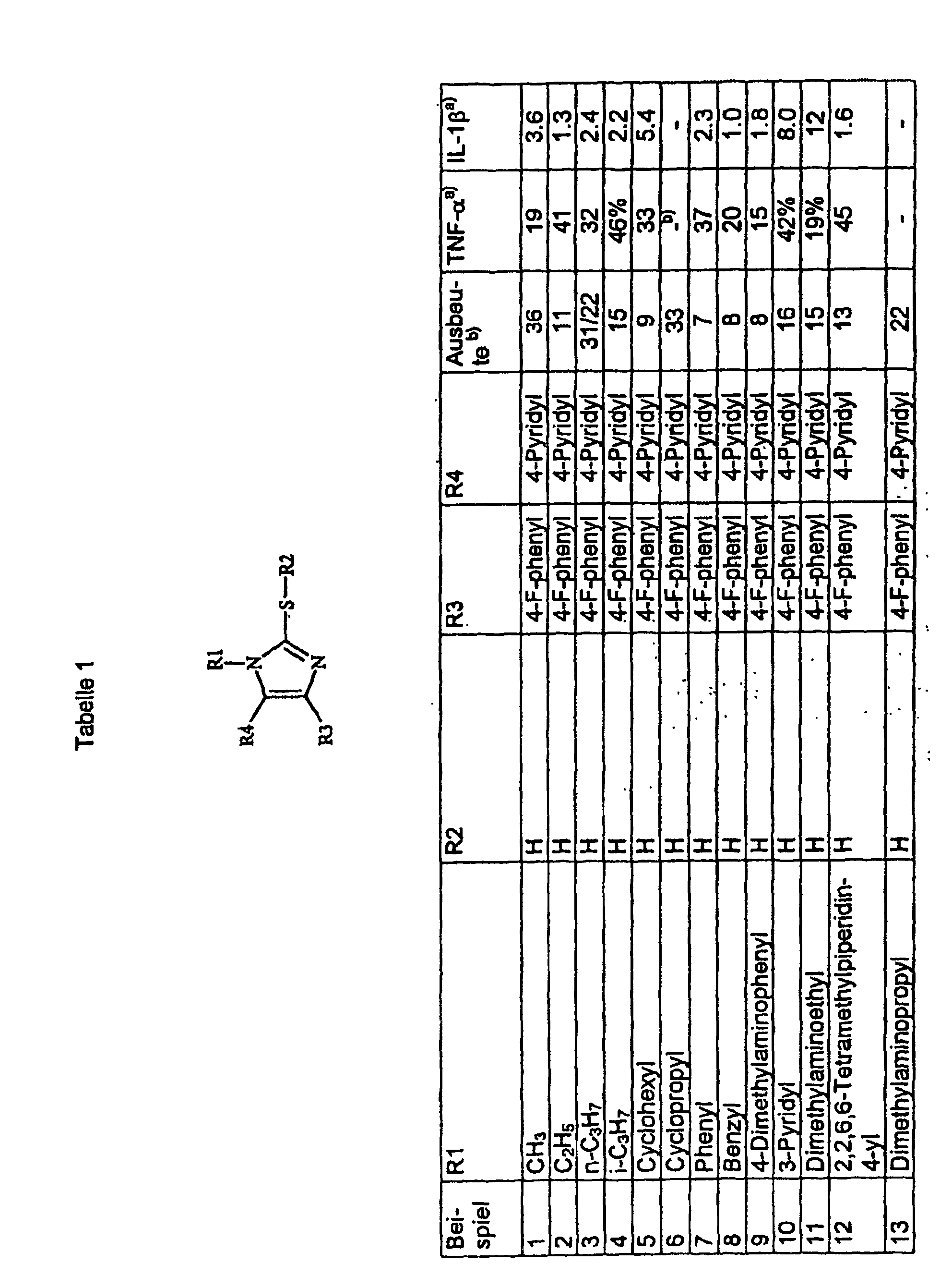

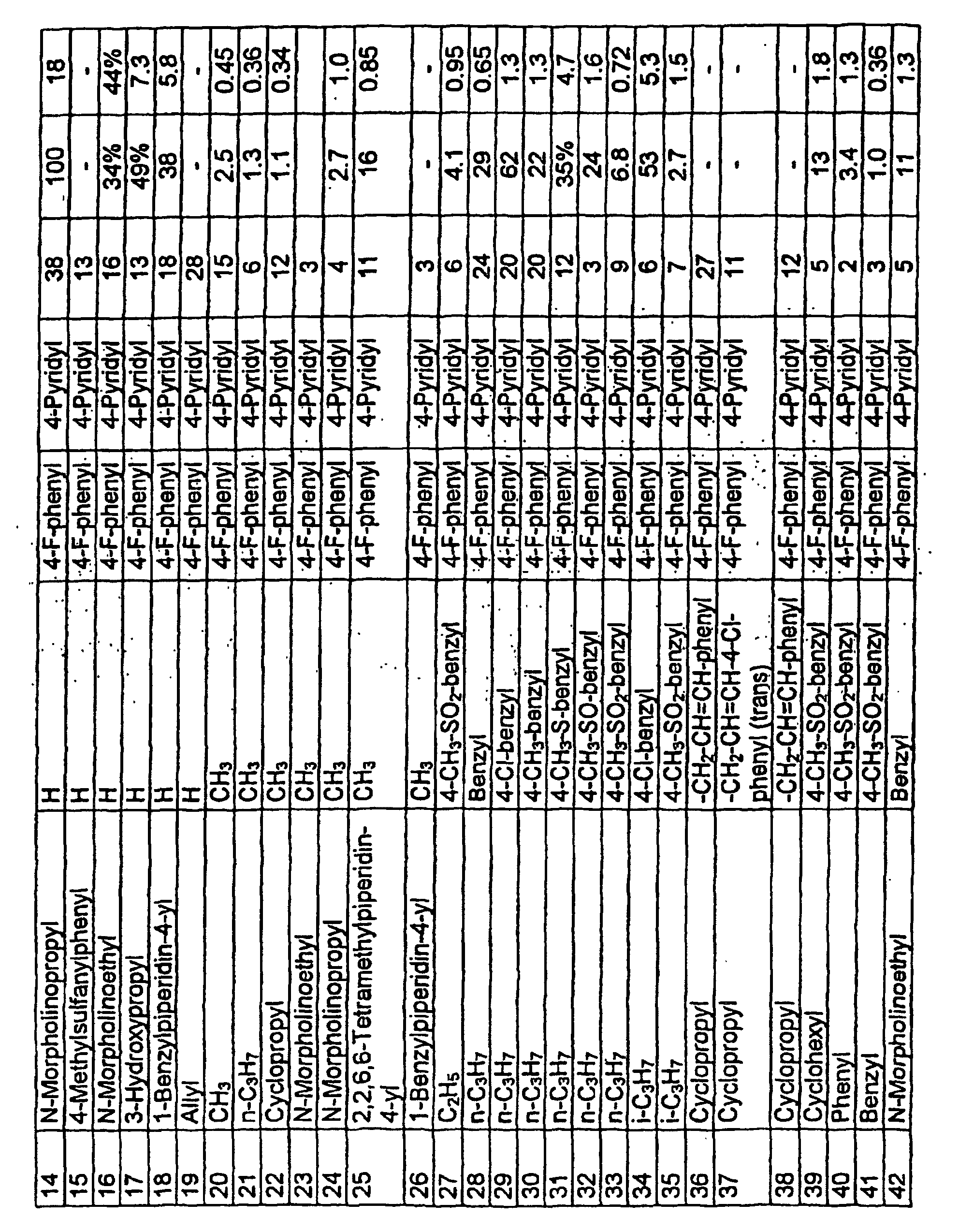

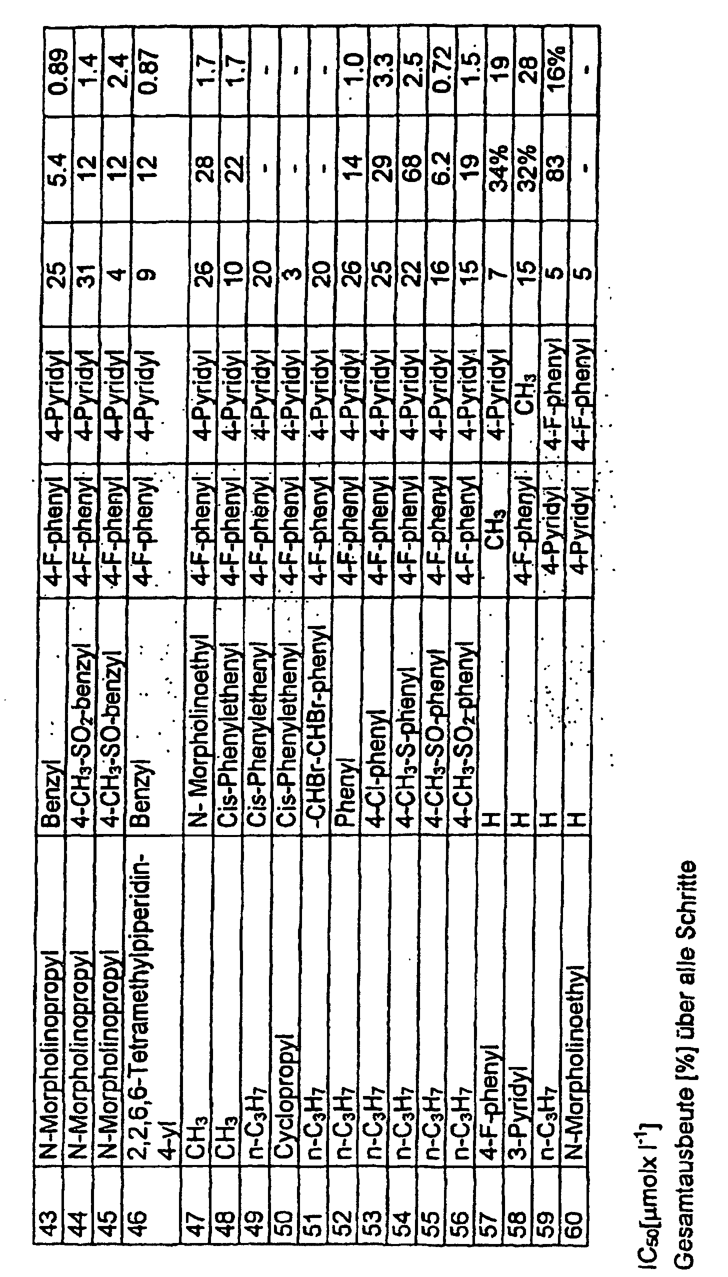

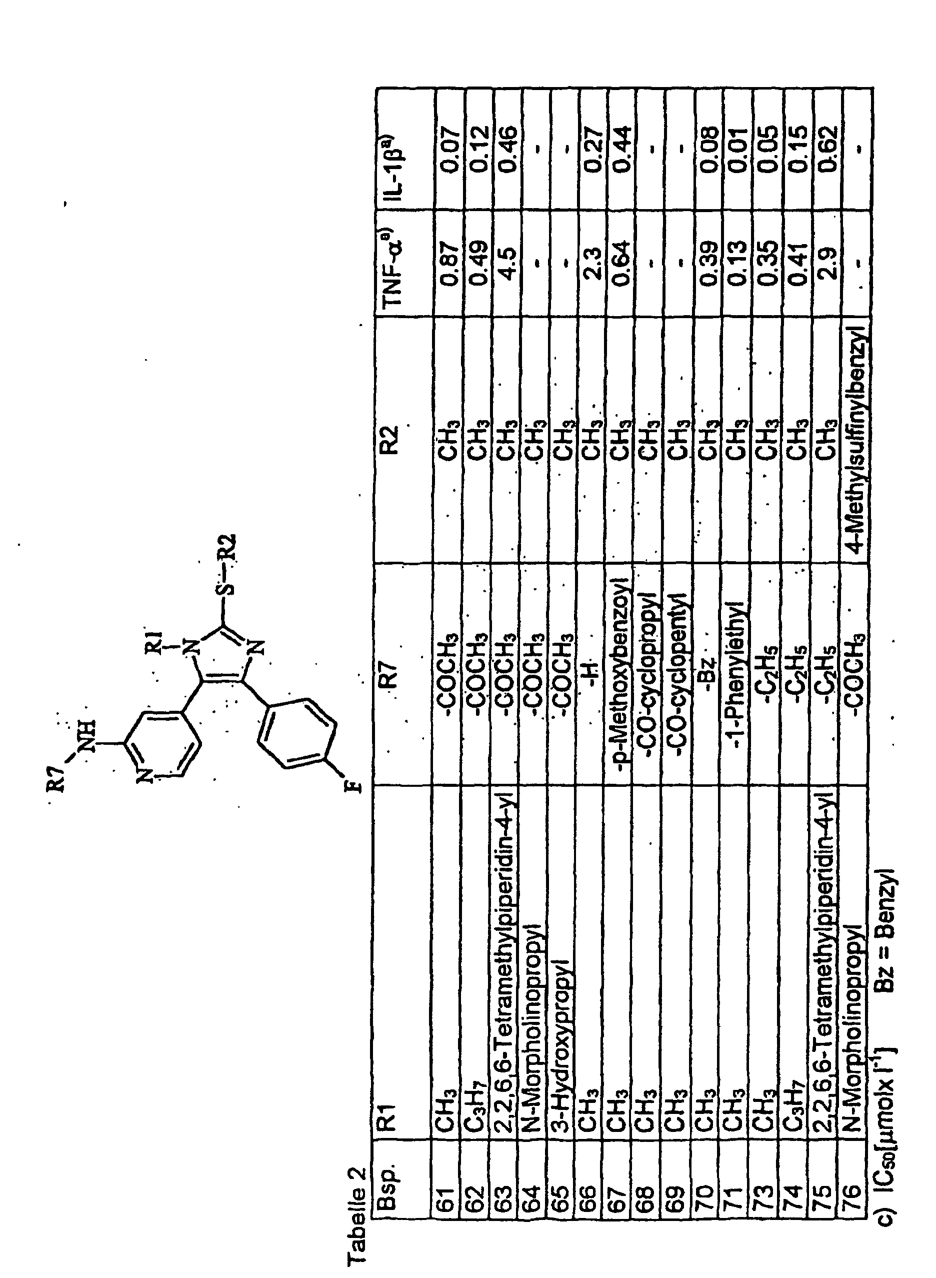

Die erfindungsgemäßen Verbindungen und die Verfahren zu ihrer Herstellung werden nun an nachstehenden Beispielen näher beschrieben, ohne die Erfindung zu beschränken.

### Beispiele

### Beispiel 1: 4-(4-Fluorphenyl)-1-methyl-5-(4-pyridyl)-2-thio- imidazol

### a) 2-Cyano-2-(4-Fluorphenyl)-1-(4-pyridyl)-ethan

250 ml trockenes Ethanol wurden zu metallischem Natrium (17,3 g/0,7 mol) getropft. Dann wurden Isonicotinsäureethylester (75,8 g/ 0,5 mol) und 4-Fluorphenylacetonitril (67,6 g/ 0,5 mol) zugetropft und das Gemisch wurde anschließend 15 min unter Rückfluss erhitzt. Nach dem Abkühlen wurde der Ansatz mit 600 ml destilliertem Wasser versetzt. Beim Ansäuem auf einen pH-Wert von 1 mit konzentrierter Salzsäure (HCl) fiel die gewünschte Verbindung 2-Cyano-2-(4-fluorphenyl)-1-(4-pyridyl)-ethan als gelber Niederschlag aus. Der Niederschlag wurde abfiltriert, mit destilliertem Wasser gewaschen und im Vakuum über Phosphorpentoxid (P₂O₅) getrocknet. Die Ausbeute betrug 85,0 g (62%).

### b) 2-(4-Fluorphenyl)-1-(4-pyridyl)-ethanon

2-Cyano-2-(4-fluorphenyl)-1-(4-pyridyl)-ethan (40,6 g/0,15 mol) aus Beispiel 1a wurde in 300 ml 48%iger Bromwasserstoffsäure (HBr) suspendiert und das Reaktionsgemisch 18 h unter Rückfluss erhitzt. Nach dem Abkühlen wurde der Ansatz mit Ammoniakwasser auf einen pH-Wert von 9 eingestellt. Die dabei ausfallende im Titel benannte Verbindung wurde abfiltriert, mit destilliertem Wasser gewaschen und im Vakuum über P₂O₅ getrocknet. Die Ausbeute betrug 25,6 g (80%).

### c) 2-(4-Fluorphenyl)-1-(4-pyridyl)-α-hydroxyiminoethanon

15,0 g (0,07 mol) 2-(4-Fluorphenyl)-1-(4-pyridyl)-ethanon aus Beispiel 1b wurden in 70 ml Eisessig gelöst. Eine Lösung von 4,8 g (0,07 mol) NaNO₂ in 11 ml Wasser wurde langsam zu der Vorlage getropft und das Reaktionsgemisch bei Raumtemperatur gerührt. Nach 3 h wurden 400 ml destilliertes Wasser zugesetzt und weitere 3 h bei Raumtemperatur gerührt. Es fiel die im Titel benannte Verbindung (3) aus. Sie wurde abfiltriert, mit destilliertem Wasser gewaschen und im Vakuum über P₂O₅ getrocknet. Die Ausbeute betrug 15,2 g (90%).

### d) 4-(4-Fluorphenyl)-1-methyl-5-(4-pyridyl)-imidazol-N-oxid

2,0 g 2-(4-Fluorphenyl)-1-(4-pyridyl)-α-hydroxyiminoethanon aus vorstehendem Beispiel 1c wurden zusammen mit der doppelten äquivalenten Menge 1,3,5-Trimethylhexahydro-1,3,5-triazin in 20 ml trockenem Ethanol gelöst und 10 h unter Rückfluss erhitzt. Nach dem Abkühlen wurde das Ethanol am Rotationsverdampfer entfernt. Der teilweise ölige Rückstand verfestigte sich bei Zugabe von Diethylether. Der Niederschlag wurde abfiltriert und im Vakuum getrocknet. Die Ausbeute war 82%.

### e) 4-(4-Fluorphenyl)-1-methyl-5-(4-pyridyl)-imidazol-2-thion

0,5 g 5-(4-Fluorphenyl)-4-(4-pyridyl)-3-methyl-imidazol-N-oxid aus Beispiel 1d wurden in 20 ml CHCl₃ gelöst und das Reaktionsgemisch wurde im Eisbad abgekühlt. Eine äquimolare Lösung von 2,2,4,4-Tetramethyl-3-thiono-cyclobutanon in CHCl₃ wurde langsam zur Vorlage getropft und anschließend 30 min im Eisbad gerührt. Das Eisbad wurde entfernt und es wurde 1 h bei Raumtemperaturweitergerührt. Anschließend wurde das Lösungsmittel am Rotationverdampfer entfernt und der feste Rückstand in Diethylether ausgerührt. Der Niederschlag wurde abfiltriert und im Vakuum getrocknet. Die Ausbeute war 98%.
IR: 1/λ (cm⁻¹) = 1601, 1506, 1229, 1004, 843, 832
¹H NMR (d₆-DMSO, ppm): 12.95 (bs, 1H); 8.69-8.66 (m, 2H), 7.45-7.42 (m, 2H), 7.27-7.12 (m, 4H), 3.39 (s, 3H)

### Beispiel 2: 1-Ethyl-4-(4-fluorphenyl)-5-(4-pyridyl)-2-thio-imidazol

### a) 1-Ethyl-4-(4-fluorphenyl)-5-(4-pyridyl)-imidazol-2-on

Zunächst wurde 2-(4-Fluorphenyl)-1-(4-pyridyl)-α-hydroxyiminoethanon wie in Beispiel 1 in den Schritten (a) bis (c) beschrieben hergestellt. Anschließend wurden 4,0 g des Iminoethanons zusammen mit der äquimolaren Menge Ethylamin und der äquimolaren Menge Formaldehyd (36%-ige wässrige Lösung) 4 h unter Rückfluss erhitzt. Nach dem Abkühlen wurde das Reaktionsgemisch mit Ammoniakwasser neutralisiert und mit CH₂Cl₂ dreimal extrahiert. Die organischen Phasen wurden vereinigt und über Na₂SO₄ getrocknet. Das Trocknungsmittel wurde abfiltriert und das Lösungsmittel am Rotationsverdampfer entfernt. Der teilweise ölige Rückstand wurde durch Zugabe von Diethylether verfestigt Der Niederschlag wurde abfiltriert und im Vakuum getrocknet. Die Ausbeute war 63%.

### b) 2-Chlor-1-ethyl-4-(4-fluorphenyl)-5-(4-pyridyl)-imidazol

2,0 g 1-Ethyl-4-(4-fluorphenyl)-5-(4-pyridyl)-imidazol-2-on wurden mit 35 ml POCl₃ und einer kleinen Menge NH₄Cl versetzt und das Reaktionsgemisch 9 h unter Rückfluss erhitzt. Nach dem Abkühlen wurde das überschüssige POCl₃ weitgehend abdestilliert und der Rückstand vorsichtig mit destilliertem Wasser versetzt. Beim Neutralisieren mit 20%-iger NaOH fiel die im Titel benannte Verbindung aus. Der Niederschlag wurde abfiltriert und über P₂O₅ im Vakuum getrocknet. Ausbeute: 81%.

### c) 1-Ethyl-4-(4-fluorphenyl)-5-(4-pyridyl)-imidazol-2-thion

NaH (4,5 Äq.) wurde in 10 ml DMF suspendiert und 4-Chlorbenzylthiol (4,5 Äq.) langsam zugetropft. Das Reaktionsgemisch wurde 45 min bei Raumtemperatur gerührt. Anschießend wurden 2,0 g des in vorstehendem Schritt gewonnenen 2-Chlor-1-ethyl-4-(4-fluorphenyl)-5-(4-pyridyl)-imidazols zugegeben. Der Ansatz wurde 10 h unter Rückfluss erhitzt. Nach dem Abkühlen wurde das Gemisch mit destilliertem Wasser versetzt, mit konzentrierter HCl auf einen pH-Wert von 1 eingestellt und sechsmal mit Diethylether gewaschen. Beim Neutralisieren der Wasserphase mit 20%-iger NaOH fiel die im Titel benannte Verbindung aus. Der Niederschlag wurde abfiltriert und über P₂O₅ im Vakuum getrocknet. Die Reinigung erfolgte durch Umkristallisieren. Die Ausbeute war 50%.
IR: 1/λ (cm⁻¹) = 3059, 1587, 1498, 1220, 837, 814
¹H NMR (CDCl₃, ppm): 12.63 (bs, 1H), 8.74-8.72 (m, 2H), 7.27-7.17 (m, 4H), 7.0-6.90 (m, 2H), 4.08 (q, 2H, J= 7.1 Hz), 1.21 (t, 3H, J= 7.1 Hz)

### Beispiel 3A: 4-(4-Fluorphenyl)-1-n-propyl-5-(4-pyridyl)-2-thio-imidazol

Es wurde das Verfahren gemäß Beispiel 1 verwendet, wobei das 2-(4-Fluorphenyl)-1-(4-pyridyl)-α-hydroxyiminoethanon in Schritt (d) mit der doppelten äquivalenten Menge 1,3,5-Tri-n-propylhexahydro-1,3,5-triazin umgesetzt wurde.
Die Ausbeute lag im Bereich von 60-91%.
IR: 1/λ (cm⁻¹) = 2932, 1586, 1500, 1221, 831, 814
¹H NMR (CDCl₃, ppm): 12.47 (bs, 1H), 8.76-8.73 (m, 2H), 7.26-7.13 (m, 4H), 7.0-6.96 (m, 2H), 3.98 (t, 2H, J= 7.8 Hz), 1.65 (m, 2H), 0.82 (t, 3H, J= 7.4 Hz)

### Beispiel 3B: 4-(4-Fluorphenyl)-1-n-propyl-5-(4-pyridyl)-2-thio-imidazol

Alternativ wurde zur Herstellung der im Titel benannten Verbindung das Verfahren gemäß Beispiel 2 verwendet, wobei 2-(4-Fluorphenyl)-1-(4-pyridyl)-α-hydroxyiminoethanon in Schritt (a) mit der äquimolaren Menge n-Propylamin umgesetzt wurde.
Die Ausbeute lag im Bereich von 32-72%.

### Beispiel 4: 4-(4-Fluorphenyl)-1-isopropyl-5-(4-pyridyl)-2-thio-imidazol

Zur Herstellung der im Titel benannten Verbindung wurde das Verfahren gemäß Beispiel 2 verwendet, wobei 2-(4-Fluorphenyl)-1-(4-pyridyl)-α-hydroxyiminoethanon in Schritt (a) zusammen mit der äquimolaren Menge Isopropylamin umgesetzt wurde.
IR: 1/λ (cm⁻¹) = 30.40, 1584, 1500, 1230, 841, 819
¹H NMR (CDCl₃, ppm): 11.73 (bs, 1H), 8.76-8.74 (m, 2H,), 7.28 (m, 2H), 7.17-7.10 (m, 2H), 7.0-6.92 (m, 2H), 4.89 (m, 1H), 1.48 (s, 3H), 1.45 (s, 3H)

### Beispiel 5: 1-Cyclohexyl-4-(4-fluorphenyl)-5-(4-pyridyl)-2-thio-imidazol

Zur Herstellung der im Titel benannten Verbindung wurde das Verfahren gemäß Beispiel 2 verwendet, wobei 2-(4-Fluorphenyl)-1-(4-pyridyl)-α-hydroxyiminoethanon in Schritt (a) zusammen mit der äquimolaren Menge Cyclohexylamin umgesetzt wurde.
IR: 1/λ (cm⁻¹) = 2934, 1560, 1505, 1228, 842
¹H NMR (CDCl₃, ppm): 11.32 (bs, 1H), 8.76-8.73 (m, 2H), 7.30-7.31 (m, 2H), 7.15-7.08 (m,2H), 7.01-6.92 (m, 2H), 4.60-4.25 (m, 1H), 2.0-1.18 (m, 10H)

### Beispiel 6: 1-Cyclopropyl-4-(4-fluorphenyl)-5-(4-pyridyl)-2-thio-imidazol

Es wurde das gleiche Verfahren wie in Beispiel 1 verwendet, wobei das 2-(4-Fluorphenyl)-1-(4-pyridyl)-α-hydroxylminoethanon in Schritt (d) mit der doppelten äquivalenten Menge 1,3,5-Tricyclopropylhexahydro-1,3,5-triazin umgesetzt wurde. IR: 1/λ (cm⁻¹) = 3013, 1589, 1515, 1499, 1487, 1223, 830, 685
¹H NMR (CDCl₃, ppm): 12.76 (bs, 1H), 8.68-8.65 (m, 2H), 7.26-7.19 (m, 4H), 7.07-6.99 (m, 2H), 3.12-3.08 (m, 1H), 1.02-0.95 (m, 2H), 0.76-0.71 (m, 2H)

### Beispiel 7: 4-(4-Fluorphenyl)-1-phenyl-5-(4-pyridyl)-2-thio-imidazol

Zur Herstellung der im Titel benannten Verbindung wurde das Verfahren gemäß Beispiel 2 verwendet, wobei 2-(4-Fluorphenyl)-1-(4-pyridyl)-α-hydroxyiminoethanon in Schritt (a) zusammen mit der äquimolaren Menge Anilin umgesetzt wurde.
IR: 1/λ (cm⁻¹) = 2880, 1597, 1504, 1227, 844, 825
¹H NMR (CDCl₃; ppm): 11.58 (bs, 1H), 8.48-8.41 (m, 2H), 7.78-6.74 (m, 11H)

### Beispiel 8: 1-Benzyl-4-(4-fluorphenyl)-5-(4-pyridyl)-2-thio-imidazol

Zur Herstellung der im Titel benannten Verbindung wurde das Verfahren gemäß Beispiel 2 verwendet, wobei 2-(4-Fluorphenyl)-1-(4-pyridyl)-α-hydroxyiminoethanon in Schritt (a) zusammen mit der äquimolaren Menge Benzylamin umgesetzt wurde. IR: 1/λ (cm⁻¹) = 3032, 1587, 1497, 1225, 1158, 837, 816
¹H NMR (CDCl3, ppm): 12.88 (bs, 1H), 8.56-8.53 (m, 2H), 7.27-6.90 (m, 11H), 5.28 (s, 2H)

### Beispiel 9: 1-Dimethylaminophenyl-4-(4-fluorphenyl)-5-(4-pyridyl)-2-thio-imidazol

Zur Herstellung der im Titel benannten Verbindung wurde das Verfahren gemäß Beispiel 2 verwendet, wobei 2-(4-Fluorphenyl)-1-(4-pyridyl)-α-hydroxyiminoethanon in Schritt (a) zusammen mit der äquimolaren Menge 4-Dimethylaminobenzylamin umgesetzt wurde.
IR: 1/λ (cm⁻¹) = 2891, 1606, 1500, 1357, 1225, 835, 816
¹H NMR (d₆-DMSO, ppm): 13.05 (bs, 1H), 8.43-8.41 (m, 2H), 7.37-7.03 (m, 8H), 6.98-6.60 (m, 2H), 2.89 (s, 6H)

### Beispiel 10: 4-(4-Fluorphenyl)-1-(3-pyridyl)-5-(4-pyridyl)-2-thio-imidazol

Zur Herstellung der im Titel benannten Verbindung wurde das Verfahren gemäß Beispiel 2 verwendet, wobei 2-(4-Fluorphenyl)-1-(4-pyridyl)-α-hydroxyiminoethanon in Schritt (a) zusammen mit der äquimolaren Menge 3-Pyridylamin umgesetzt wurde. IR: 1/λ (cm⁻¹) = 3035, 1597, 1478, 1433, 1433, 1224, 813, 708
¹H NMR (d₆-DMSO, ppm): 13.34 (s, 1H), 8.54-8.45 (m, 4H), 7.76-7.75 (m, 1H), 7.40-7.13 (m, 7H)

### Beispiel 11: 1-Dimethylaminoethyl-4-(4-fluorphenyl)-5-(4-pyridyl)-2-thio-imidazol

Es wurde das gleiche Verfahren wie in Beispiel 1 verwendet, wobei das 2-(4-Fluorphenyl)-1-(4-pyridyl)-α-hydroxyiminoethanon in Schritt (d) mit der doppelten äquivalenten Menge 1,3,5-Tri(2-dimethylaminoethyl)hexahydro-1,3,5-triazin umgesetzt wurde.
IR: 1/λ (cm⁻¹) = 2772, 1597, 1503, 1225, 835, 815
¹H NMR (CDCl₃, ppm): 8.74-8.72 (m, 2H), 7.30-7.17 (m, 4H), 7.04-6.94 (m, 2H), 4.13 (t, 2H, J= 6.8 Hz), 2.56 (t, 2H, J= 6.7 Hz), 2.11 (s, 6H)

### Beispiel 12: 4-(4-Fluorphenyl)-5-(4-pyridyl)-1-(2,2,6,6-tetramethylpiperidin-4-yl)-2-thio-imidazol

Es wurde das gleiche Verfahren wie in Beispiel 1 verwendet, wobei das 2-(4-Fluorphenyl)-1-(4-pyridyl)-α-hydroxyiminoethanon in Schritt (d) mit der doppelten molaren Menge 2,2,6,6-Tetramethyl-4-methylenamino-piperidin umgesetzt wurde.
IR: 1/λ (cm⁻¹) = 2964, 1587, 1498, 1352, 1234, 838, 815

### Beispiel 13 : 1-Dimethylaminopropyl-4-(4-fluorphenyl)-5-(4-pyridyl)-2-thio-imidazol

Es wurde das gleiche Verfahren wie in Beispiel 1 verwendet, wobei das 2-(4-Fluorphenyl)-1-(4-pyridyl)-α-hydroxyiminoethanon in Schritt (d) mit der doppelten äquivalenten Menge 1,3,5-Tri(3-dimethylaminopropyl)hexahydro-1,3,5-triazin wurde.

### Beispiel 14: 4-(4-Fluorphenyl)-1-(3-N-morpholinopropyl)-5-(4-pyridyl)-2-thio-imidazol

Es wurde das gleiche Verfahren wie in Beispiel 1 verwendet, wobei das 2-(4-Fluorphenyl)-1-(4-pyridyl)-α-hydroxyiminoethanon in Schritt (d) mit der doppelten äquivalenten Menge 1,3,5-Tri-(N-morpholinopropyl)hexahydro-1,3,5-triazin umgesetzt wurde.
IR: 1/λ (cm⁻¹) = 2847, 1502, 1233, 1114, 842, 817
¹H NMR (CDCl₃,ppm): 12.11 (bs, 1H), 8.75-8.71 (m, 2H), 7.26-7.18 (m, 4H), 7.05-6.95 (m, 2H), 4.15-4.07 (m, 2H), 3.61-3.57 (m, 4H), 2.32-2.23 (m, 6H),1.86-1.75 (m, 2H)

### Beispiel 15: 4-(4-Fluorphenyl)-1-(4-methylsulfanylphenyl)-5-(4-pyridyl)-2-thio-imidazol

Es wurde das gleiche Verfahren wie in Beispiel 1 verwendet, wobei das 2-(4-Fluorphenyl)-1-(4-pyridyl)-α-hydroxyiminoethanon in Schritt (d) mit der doppelten äquivalenten Menge 1,3,5-Tri(4-methylsulfanylphenyl)hexahydro-1,3,5-triazin umgesetzt wurde.
IR: 1/λ (cm⁻¹) = 2693, 1597, 1495, 1220, 844, 817
¹H NMR (CDCl₃, ppm): 12.43 (bs, 1H), 8.47-8.44 (m, 2H), 7.32-7.12 (m, 6H), 7.06-6.97 (m, 2H), 6.90-6.87 (m, 2H), 2.50 (s, 3H)

### Beispiel 16: 4-(4-Fluorphenyl)-1-N-morpholinoethyl-5-(4-pyridyl)-2-thio-imidazol

Es wurde das gleiche Verfahren wie in Beispiel 1 verwendet, wober das 2-(4-Fluorphenyl)-1-(4-pyridyl)-α-hydroxyiminoethanon in Schritt (d) mit der doppelten äquivalenten Menge 1,3,5-Tri(N-morpholinoethylhexahydro-1,3,5-triazin umgesetzt wurde.
IR: 1/λ (cm⁻¹) = 2813, 1599, 1508, 1232, 1117, 850, 835
¹H NMR (d₆-DMSO): 12.91 (bs, 1H), 8.71-8.68 (m, 2H), 7.49-7.46 (m, 2H), 7.25-7.16 (m, 4H), 4.04 (t, 2H, J= Hz), 2.40 (t, 2H, J= Hz), 2.16 (t, 4H, J= 3.8 Hz)

### Beispiel 17: 4-(4-Fluorphenyl)-1-(3-hydroxypropyl)-5-(4-pyridyl)-2-thio-imidazol

Es wurde das gleiche Verfahren wie in Beispiel 1 verwendet, wobei das 2-(4-Fluorphenyl)-1-(4-pyridyl)-α-hydroxyiminoethanon in Schritt (d) mit der doppelten äquivalenten Menge 1,3,5-Tri(3-hydroxypropyl)hexahydro-1,3,5-triazin umgesetzt wurde.
IR: 1/λ (cm⁻¹) = 3049, 2926, 1499, 1223, 1162, 1061, 838
¹H NMR (d₆-DMSO, ppm): 12.98 (s, 1H), 8.71-8.68 (m,2H), 7.47-7.44 (m, 2H), 7.29-7.12 (m, 4H), 4.47-4.43 (bs, 1H), 3.97 (t, 2H, J= 7.4 Hz), 3.27 (t, 2H, J= 6.2 Hz), 1.68-1.54 (m, 2H)

### Beispiel 18: 1-(1-Benzylpiperidin-4-yl)-4-(4-fluorphenyl)-5-(4-pyridyl)-2-thio-imidazol

Es wurde das gleiche Verfahren wie in Beispiel 1 verwendet, wobei das 2-(4-Fluorphenyl)-1-(4-pyridyl)-α-hydroxylminoethanon in Schritt (d) mit der doppelten molaren Menge 1-Benzyl-4-methylenamino-piperidin umgesetzt wurde.
IR: 1/λ (cm⁻¹) = 2903, 1504, 1247, 1227, 853, 741
¹H NMR (d₆-DMSO): 12.93 (s, 1H), 8.73-8.70 (m, 2H), 7.50-7.47 (m, 2H), 7.29-7.11 (m, 9H), 3.96-4.12 (m, 1H), 3.38 (s, 2H), 3.85-3.75 (m, 2H), 2.31-2.18 (m, 2H), 1.93-1.64 (m, 4H)

### Beispiel 19: 1-Allyl-4-(4-fluorphenyl)-5-(4-pyridyl)-2-thio-imidazol

Es wurde das gleiche Verfahren wie in Beispiel 1 verwendet, wobei das 2-(4-Fluorphenyl)-1-(4-pyridyl)-α-hydroxyiminoethanon in Schritt (d) mit der doppelten äquivalenten Menge 1,3,5-Tri(prop-2-en-1-yl)hexahydro-1,3,5-triazin umgesetzt wurde.
IR: 1/λ (cm⁻¹) = 2695, 1700, 1600, 1506, 1421, 1227, 1005, 934, 927, 841, 829, 817 ¹H NMR (CDCl₃, ppm): 12.49 (bs, 1H), 8.72-8.65 (m, 2H), 7.29-7.22 (m, 4H), 7.04-6.96 (m, 2H), 6.00-5.81 (m, 1H), 5.25-5.19 (m, 1H), 5.02-4.93 (m, 1H), 4.66-4.64 (m, 2H)

### Beispiel 20: 4-(4-Fluorphenyl)-1-methyl-2-methylthio-5-(4-pyridyl-imidazol

a) Die Herstellung des 1-substituierte 4-(4-Fluorphenyl)-5-(4-pyridyl)-imidazol-2-thions (Verbindung 5 aus Schema 1) erfolgte wie in Beispiel 1 beschrieben, wobei die zugegebene Iminverbindung 1,3,5-Trimethylhexahydro-1,3,5-triazin war.
b) Anschließend wurden 0,7 g der gewonnenen Thionverbindung (5) zur Methylierung des Schwefels unter Schutzgas in 20 ml trockenem Ethanol suspendiert und mit der äquimolaren Menge an Dimethylsulfat oder Methyliodid versetzt. Nach Zugabe einer Spatelspitze Na₂CO₃ wurde das Reaktionsgemisch 3 h unter Rückfluss erhitzt. Nach dem Abkühlen wurden die anorganischen Salze abfiltriert und das Lösungsmittel am Rotationsverdampfer entfernt. Das Rohprodukt wurde mittels Säulenchromatographie gereinigt.
   Die Ausbeute war 43%.
   IR: 1/λ (cm⁻¹) = 1603, 1510, 1220, 1160, 850, 830, 814
   ¹H NMR (CDCl₃, ppm): 8.70-8.67 (m, 2H), 7.43-7.36 (m, 2H), 7.24-7.22 (m, 2H), 6.98-6.89 (m, 2H), 3.47 (s, 3H), 2.72 (s, 3H)

### Beispiel 21: 4-(4-Fluorphenyl)-2-methylthio-1-n-propyl-5-(4-pyridyl)-imidazol

Die im Titel benannte Verbindung wurde analog zu dem Verfahren aus Beispiel 20 hergestellt. Für den Ringschluss der Imidazolverbindung wurde 1,3,5-Tri-n-propylhexahydro-1,3,5-triazin eingesetzt.
IR: 1/λ (cm⁻¹) = 2929, 1601, 1511, 1221, 849, 829, 816
¹H NMR (CDCl₃, ppm): 8.71- 8.68 (m, 2H), 7.41-7.34 (m, 2H), 7.26-7.23 (m, 2H), 6.96-6.87 (m, 2H), 3.79 (t, 2H, J= 7.7 Hz), 2.74 (s, 3H), 1.64-1.52 (m, 2H), 0.80 (t, 3H, J= 7.4 Hz)

### Beispiel 22: 1-Cyclopropyl-4-(4-fluorphenyl)-2-methylthio-5-(4-pyridyl)-imidazol

Die im Titel benannte Verbindung wurde analog zu dem Verfahren aus Beispiel 20 hergestellt. Für den Ringschluss der Imidazolverbindung wurde 1,3,5-Tricyclopropylhexahydro-1,3,5-triazin eingesetzt.
¹H NMR (CDCl₃, ppm): 8.64-8.61 (m, 2H), 7.41-7:34 (m, 2H), 7.27-7.24 (m, 2H), 6.98-6.90 (m, 2H), 3.13-3.02 (m, 1H), 2.74 (s, 3H); 0.95-0.91 (m; 2H), 0.70-0.66 (m, 2H)

### Beispiel 23: 4-(4-Fluorphenyl)-2-methylthio-1-N-morpholinoethyl-5-(4-pyridyl)-imidazol

Die im Titel benannte Verbindung wurde analog zu dem Verfahren aus Beispiel 20 hergestellt. Für den Ringschluss der Imidazolverbindung wurde 1,3,5-Tri(N-morpholino)ethylhexahydro-1,3,5-triazinethanamin eingesetzt.
IR: 1/λ (cm⁻¹) = 2852, 1600, 1509, 1215, 1114, 671, 841, 813
¹H NMR (CDCl₃, ppm): 8.71-8.68 (m, 2H), 7.41-7.34 (m, 2H), 7.30 (m, 2H), 6.96-6.87 (m, 2H), 3.96 (t, 3H, J= 7.0 Hz), 3.60 (t, 4H, J= 4.6 Hz), 2.74 (s, 3H), 2.46 (t, 2H, J= 7.0 Hz), 2.32 (t, 4H, J= 4.7 Hz)

### Beispiel 24: 4-(4-Fluorphenyl)-2-methylthio-1-N-morpholinopropyl-5-(4-pyridyl)-imidazol

Die im Titel benannte Verbindung wurde analog zu dem Verfahren aus Beispiel 20 hergestellt. Für den Ringschluss der Imidazolverbindung wurde 1,3,5-Tri(3-N-morpholinopropyl)hexahydro-1,3,5-triazin eingesetzt.
IR: 1/λ (cm⁻¹) = 2814, 1509, 1219, 1114, 842
¹H NMR (CDCl₃, ppm): 8.71-8.68 (m, 2H), 7.41-7.36 (m, 2H), 7.27-7.23 (m, 2H), 6.96-6.87 (m, 2H), 3.98-90 (m, 2H), 3.64-3.59 (m, 4H), 2.74 (s, 3H), 2.27-2.19 (m, 6H), 1.77-1.68 (m, 2H)

### Beispiel 25: 4-(4-Fluorphenyl)-2-methylthio-5-(4-pyridyl)-1-(2,2,6,6-tetramethylpiperidin-4-yl)-imidazol

Die im Titel benannte Verbindung wurde analog zu dem Verfahren aus Beispiel 20 hergestellt. Für den Ringschluss der Imidazolverbindung wurde 2,2,6,6-Tetramethyl-4-methylenamino-piperidin eingesetzt.
IR: 1/λ (cm⁻¹) = 2968, 1600, 1509, 1343, 1229, 1033, 835, 813
¹H NMR (CDCl₃, ppm): 8.73-8.70 (m, 2H), 7.37-7.22 (m, 4H), 6.94-6.86 (m, 2H), 4.41-4.28 (m, 1H); 2.75 (s, 3H), 2.05-2.04 (m, 2H), 1.74-1.66 (m, 2H), 1.16, 1.04 (2s, 12H)

### Beispiel 26: 1-Benzylpiperidin-4-yl-4-(4-fluorphenyl)-2-methylthio-5-(4-pyridyl)-imidazol

Die im Titel benannte Verbindung wurde analog zu dem Verfahren aus Beispiel 20 hergestellt. Für den Ringschluss der Imidazolverbindung wurde 1-Benzyl-4-methylenamino-piperidin eingesetzt.
IR: 1/λ (cm⁻¹) = 2929, 2809, 1602, 1509, 1220, 1158, 840, 828, 814, 743, 701
¹H NMR (d₆-DMSO, ppm): 8.72-8.69 (m, 2H), 7.41-7.38 (m, 2H), 7.30-7.21 (m, 7H), 7.09-7.0 (m, 2H), 3.60-3.72 (m, 1H), 3.40 (s, 1H), 2.85-2.80 (m, 2H), 2.42-2.22 (m, 2H), 1.81-1.76 (m, 4H)

### Beispiel 27: 1-Ethyl-4-(4-fluorphenyl)-2-(4-methylsulfonyl)benzylthio-5-(4-pyridyl)-imidazol

a) Die Herstellung des 1-substituierten 4-(4-Fluorphenyl)-5-(4-pyridyl)-imidazol-2-thions (Verbindung 5 aus Schema 2) erfolgte wie in Beispiel 2 beschrieben, wobei die zugegebene Aminverbindung Ethylamin war.
b) o,4 g der gewonnenen Thionverbindung (5) wurden zur Benzylierung des Schwefels unter Schutzgas in 15 ml trockenem Ethanol suspendiert und mit der äquimolaren Menge 4-Methylsulfonyl-benzylchlorid versetzt. Nach Zugabe einer Spatelspitze Na₂CO₃ wurde das Reaktionsgemisch 5 h unter Rückfluss erhitzt. Nach dem Abkühlen wurde das Na₂CO₃ abfiltriert und das Lösungsmittel am Rotationsverdampfer entfernt. Das Rohprodukt der im Titel benannten Verbindung (Verbindung 9 in Schema 3.2) wurde mittels Säulenchromatographie gereinigt.
   Ausbeute: 55%
   IR: 1/λ (cm⁻¹) = 1510, 1304, 1149, 841, 766
   ¹H NMR (CDCl₃, ppm): 8.71-8.69 (m, 2H), 7.91-7.87 (m, 2H), 7.62-7.58 (m, 2H), 7.41-7.34 (m, 2H), 7.26-7.21 (m, 2H), 6.98-6.91 (m, 2H), 4.52 (s, 2H), 3.73 (q, 2H, J= 7.2 Hz), 3.05 (s, 3H), 1.07 (t, 3H, J= 7.2 Hz)

### Beispiel 28: 2-Benzylthio-4-(4-fluorphenyl)-1-n-propyl-5-(4-pyridyl)-imidazol

a) Die Herstellung des 1-substituierten 4-(4-Fluorphenyl)-5-(4-pyridyl)-imidazol-2-thions (Verbindung 5 aus Schema 1) erfolgte wie in Beispiel 1 beschrieben, wobei die zugegebene Iminverbindung 1,3,5-Tri-n-propylhexahydro-1,3,5-triazin war.
b) Die Benzylierung des Schwefels erfolgte mit Benzylchlorid nach dem Verfahren aus Beispiel 27 (Schritt b).
   IR: 1/λ (cm⁻¹) = 1602, 1510, 1220, 851, 833, 815, 695
   ¹H NMR (CDCl3, ppm): 8.70-8.67 (m, 2H), 7.43-7.26 (m, 6H), 7.19-7.16 (m, 2H), 6.98-6.89 (m, 2H), 4.39 (s, 2H), 3.56 (t, 2H, J= 7.6Hz), 1. 42-1.30 (m, 2H), 0.66 (t, 3H, J= 7.4 Hz)

### Beispiel 29: 2-(4-Chlorbenzyl)thio-4-(4-fluorphenyl)-1-n-propyl-5-(4-pyridyl)-imidazol

Die im Titel benannte Verbindung wurde wie in Beispiel 28 beschrieben hergestellt, außer dass zur Benzylierung 4-Chlor-benzylchlorid verwendet wurde.
IR: 1/λ (cm⁻¹) = 2972, 1602, 1509, 1343, 1222, 1092, 844, 828, 816, 743, 698
¹H NMR (CDCl₃, ppm): 8.71-8.68 (m, 2H), 7.41-7.34 (m, 2H), 7.27-7.26 (m, 4H), 7.19-7.16 (m, 2H), 6.98-6.89 (m, 2H), 4.38 (s, 2H), 3.61 (t, 2H, J= 7.6 Hz), 1.45-1.33 (m, 2H), 0.68 (t, 3H, 7.4 Hz)

### Beispiel 30: 4-(4-Fluorphenyl)-2-(4-methylbenzyl)thio-1-n-propyl-5-(4-pyridyl)-imidazol

Die im Titel benannte Verbindung wurde wie in Beispiel 28 beschrieben hergestellt, außer dass zur Benzylierung 4-Methyl-benzylchlorid verwendet wurde.
IR: 1/λ (cm⁻¹) = 2927, 1603, 1510, 1222, 849, 831, 815
¹H NMR (CDCl₃, ppm): 8.70-8.67 (m, 2H), 7.43-7.36 (m, 2H), 7.23-7.09 (m, 6H), 6.98-6.89 (m, 2H), 4.37 (s, 2H), 3.59 (t, 2H, J= 7.7 Hz), 2.34 (s, 3H), 1.44-1.33 (m, 2H), 0.67 (t, 3H, J= 7.4 Hz)

### Beispiel 31: 4-(4-Fluorphenyl)-2-(4-methylthio)benzylthio-1-n-propyl-5-(4-pyridyl)-imidazol

Die im Titel benannte Verbindung wurde nach dem Verfahren aus Beispiel 27 hergestellt, wobei in Schritt (a) n-Propylamin und in Schritt (b) zur Benzylierung 4-Methylthiobenzylchlorid eingesetzt wurde.
IR: 1/λ (cm⁻¹) = 2922, 1602, 1508, 1405, 1222, 848, 814
¹H NMR (CDCl₃, ppm): 8.70-8.67 (m, 2H), 7.42-7.35 (m, 2H), 7.24-7.15 (m, 6H), 6.98-6.89 (m, 2H), 4.37 (s, 2H), 3.60 (t, 2H, J= 7.6 Hz), 2.48 (s, 3H), 1.44-1.33 (m, 2H), 0.68 (t, 3H, J= 7.4 Hz)

### Beispiel 32: 4-(4-Fluorphenyl)-2-(4-methylsulfinyl)benzylthio-1-n-propyl-5-(4-pyridyl)-imidazol

Es wurde das Verfahren aus Beispiel 31 wiederholt, außer dass in Schritt (b) zur Benzylierung 4-Methylsulfinyl-benzylchlorid eingesetzt wurde.
IR: 1/λ (cm⁻¹) = 2959, 1602, 1509, 1407, 1221, 1089, 1048, 842, 816
¹H NMR (CDCl₃, ppm): 8.71-8.68 (m, 2H), 7.63-7.51 (m, 4H), 7.41-7.34 (m, 2H), 7.21-7.18 (m, 2H), 6.98-6.90 (m, 2H), 4.48 (s, 2H), 3.64 (t, 2H, J= 7.6 Hz), 2.72 (s, 3H), 1.42-1.35 (m, 2H), 0.69 (t, 3H, J= 7.4 Hz)

### Beispiel 33: 4-(4-Fluorphenyl)-2-(4-methylsulfonyl)benzylthio-1-n-propyl-5-(4-pyridyl)-imidazol

Es wurde das Verfahren aus Beispiel 31 wiederholt, außer dass in Schritt (b) zur Benzylierung 4-Methylsulfonyl-benzylchlorid eingesetzt wurde.
IR: 1/λ (cm⁻¹) = 1509, 1306, 1219, 1150, 964, 844, 768, 744
¹H NMR (CDCl₃, ppm): 8.70-8.68 (m, 2H), 7.91-7.87 (m, 2H), 7.62-7.58 (m, 2H), 7.41-7.26 (m, 4H), 7.0-6.92 (m, 2H), 4.55 (s, 2H), 3.66 (t, 2H, J= 7.6 Hz), 3.05 (s, 3H), 1.48-1.37 (m, 2H), 0.71 (t, 3H, J= 7.5 Hz)

### Beispiel 34: 2-(4-Chlorbenzyl)thio-4-(4-fluorphenyl)-1-isopropyl-5-(4-pyridyl)-imidazol

Die im Titel benannte Verbindung wurde nach dem Verfahren aus Beispiel 27 hergestellt, wobei in Schritt (a) Isopropylamin und in Schritt (b) zur Benzylierung 4-Chlorbenzylchlorid eingesetzt wurde.
IR: 1/λ (cm⁻¹) = 1509, 1222, 1092, 850, 815
¹H NMR (CDCl₃, ppm): 8.73-8.71 (m, 2H), 7.38-7.21 (m, 8H), 6.97-6.88 (m, 2H), 4.49 (s, 2H), 4.27-4.20 (m, 1H), 1.38 (s, 3H), 1.27 (s, 3H)

### Beispiel 35: 4-(4-Fluorphenyl)-2-(4-methylsulfonyl)benzylthio-1-isopropyl-5-(4-pyridyl)-imidazol

Die im Titel benannte Verbindung wurde nach dem Verfahren aus Beispiel 27 hergestellt, wobei in Schritt (a) Isopropylamin und in Schritt (b) zur Benzylierung 4-Methylsulfonyl-benzylchlorid eingesetzt wurde.
IR: 1/λ (cm⁻¹) = 1510, 1306, 1219, 1149, 843, 766, 744
¹H NMR (CDCl₃, ppm): 8.72-8.69 (m, 2H), 7.92-7.88 (m, 2H), 7.67-7.62 (m, 2H), 7.35-7.21 (m, 4H), 4.60 (s, 2H), 4.25-4.18 (m, 1H), 3.05 (s, 3H), 1.39, 1.35 (2s, 6H)

### Beispiel 36: 1-Cyclopropyl-4-(4-fluorphenyl)-2-(1-phenylpropinyl)thio-5-(4-pyridyl)-imidazol

Die im Titel benannte Verbindung wurde nach dem Verfahren aus Beispiel 28 hergestellt, wobei In Schritt (a) 1,3,5-Tricyclopropylhexahydro-1,3,5-triazin und in Schritt (b) zur Benzylierung 1-Phenylprop-1-inylchlorid eingesetzt wurde.
IR: 1/λ (cm⁻¹) = 1603, 1509, 1388, 1221, 842, 816, 753, 688
¹H NMR (CDCl₃, ppm): 8.64-8.61 (m, 2H), 7.44-7.22 (m, 9H), 6.99-6.91 (m, 2H), 4.31 (s, 2H), 3.18-3.11 (m, 1H), 0.97-0.90 (m, 2H), 0.75-0.69 (m, 2H)

### Beispiel 37: 1-Cyclopropyl-4-(4-fluorphenyl)-2-(1-(4-chlor)phenylpropenyl)thio-5-(4-pyridyl)-imidazol

Es wurde wie in Beispiel 36 verfahren, außer dass in Schritt (b) zur Benzylierung 1-(4-Chlorphenyl)-prop-1-enylchlorid eingesetzt wurde.
¹H NMR (CDCl₃, ppm): 8.64-8.61 (m, 2H), 7.42-7.35 (m, 2H), 7.29-7.26 (m, 4 H), 7.23-7.20 (m, 2H), 7.0-6.91 (m, 2H), 6.60 (d, 1H, J= 15.7 Hz), 6.48 -6.37 (m, 1H), 4.11 (d, 2H, J= 6.5 Hz), 3.12-3.0 (m, 1H), 0.94-0.90 (m, 2H), 0.68-0.64 (m, 2H)

### Beispiel 38: 1-Cyclopropyl-4-phenyl-2-(1-(4-Fluor-phenylpropenyl)thio-5-(4-pyridyl)-imidazol

Es wurde wie in Beispiel 36 verfahren, außer dass in Schritt (b) zur Benzylierung 1-Phenyl-prop-1-enylchlorid eingesetzt wurde.
IR: 1/λ (cm⁻¹) = 3025, 1599, 1509, 1384, 1219, 963, 838, 824, 815, 750, 692
¹H NMR (CDCl₃, ppm): 8.64-8.61 (m, 2H), 7.44-7.28 (m, 7H), 7.23-7.20(m, 2H), 7.02-6.92 (m, 2H), 6.65 (d, 2H, J= 15.8 Hz), 6.51-6.40 (m, 1H), 4.13 (d, 2H, J= 6.7 Hz), 3.11-3.04 (m, 1H), 0.95-0.88 (m, 2H), 0.71-0.65 (m, 2H)

### Beispiel 39: 1-Cyclohexyl-4-(4-fluorphenyl)-2-(4-methylsulfonyl)benzylthio-5-(4-pyridyl)-imidazol

Die im Titel benannte Verbindung wurde nach dem Verfahren aus Beispiel 27 hergestellt, wobei in Schritt (a) Cyclohexylamin und in Schritt (b) zur Benzylierung 4-Methylsulfonyl-benzylchlorid eingesetzt wurde.
IR: 1/λ (cm⁻¹) = 2930, 1599, 1509, 1304, 1149, 838, 763
¹H NMR (CDCl₃, ppm): 8.73-8.70 (m, 2H), 7.92-7.88 (m, 2H), 7.67-7.63 (m, 2H), 7.34-7.21 (m, 4H), 6.97-6.88 (m, 2H), 4.64 (s, 2H), 3.73-.371 (m, 1H), 2.10-1.62 (m, 10 H)

### Beispiel 40: 4-(4-Fluorphenyl)-2-(4-methylsulfonyl)benzylthio-1-phenyl-5-(4-pyridyl)-imidazol

Es wurde wie in .Beispiel 39 verfahren, außer dass das Amin in Schritt (a) Anilin war. IR: 1/λ (cm⁻¹) = 1598, 1510, 1408, 1303, 1149, 1090, 840, 765, 694
¹H NMR (CDCl₃, ppm): 8.41-8.38 (m, 2H), 7.89-7.85 (m, 2H), 7.61-7.38 (m, 7H), 7.07-6.92 (m, 6H), 4.50 (s, 2H), 3.04 (s, 3H)

### Beispiel 41: 1-Benzyl-4-(4-fluorphenyl)-2-(4-methylsulfonyl)benzylthio-5-(4-pyridyl)-imidazol

Es wurde wie in Beispiel 39 verfahren, außer dass als Imin in Schritt (a) Benzylamin eingesetzt wurde.
IR: 1/λ (cm⁻¹) = 1600, 1509, 1304, 1220, 1147, 1090, 843, 767, 725
¹H NMR (CDCl₃, ppm): 8.57-8.54 (m, 2H), 7.90-7.85 (m, 2H), 7.58-7.54 (m, 2H), 7.45-7.38 (m, 2H), 7.26-7.23 (m, 3H), 7.08-6.97 (m, 4H), 6.80-6.79( m, 2H), 4.93 (s, 2H), 4.47 (s, 2H), 3.05 (s, 3H)

### Beispiel 42: 2-Benzylthio-4-(4-fluorphenyl)-1-N-morpholinoethyl-5-(4-pyridyl)-imidazol

Die im Titel benannte Verbindung wurde nach dem Verfahren aus Beispiel 27 hergestellt, wobei in Schritt (a) N-Morpholino-ethylamin eingesetzt wurde und in Schritt (b) zur Benzylierung Benzylchlorid.
IR: 1/λ (cm⁻¹) = 2802, 1603, 1510, 1219, 1116, 870, 836, 814, 712, 695
¹H NMR (CDCl₃, ppm): 8.71-8.68 (m, 2H), 7.44-7.37 (m, 2H), 7.30-7.26 (m, 5H), 7.26-7.19 (m, 2H), 6.99-6.90 (m, 2H), 4.37 (s, 2H), 3.80-3.54 (m,6H), 2.24-2.17 (m, 6H)

### Beispiel 43: 2-Benzylthio-4-(4-fluorphenyl)-1-N-morpholinopropyl-5-(4-pyridyl)-imidazol

Es wurde das Beispiel 28 wiederholt, außer dass die zugesetzte Iminverbindung in Schritt (a) 1,3,5-Tri(3-N-morpholinopropyl)hexahydro-1,3,5-triazin war.
IR: 1/λ (cm⁻¹) = 2814, 1602, 1509, 1460, 1218, 1114, 970, 842, 812, 696
¹H NMR (CDCl₃, ppm): 8.70-8.67 (m, 2H), 7.43-7.26 (m, 7H), 7.20-7.17 (m, 2H), 6.98-6.89 (m, 2H), 4.39 (s, 2H), 3.74-3.56 (m, 6H), 2.19-2.06 (m, 6H), 1.55-1.36 (m, 2H)

### Beispiel 44: 4-(4-Fluorphenyl)-2-(4-methylsulfonyl)benzylthio-1-N-morpholinoethyl-5-(4-pyridyl)-imidazol

Die im Titel benannte Verbindung wurde nach dem Verfahren aus Beispiel 28 hergestellt, wobei in Schritt (a) 1,3,5-Tri(3-N-morpholinopropyl)hexahydro-1,3,5-triazin und in Schritt (b) zur Benzylierung 4-Methylsulfonyl-benzylchlorid eingesetzt wurde.
IR: 1/λ (cm⁻¹) = 2924, 1600, 1510, 1302, 1147, 1115, 839, 765
¹H NMR (CDCl₃, ppm): 8.71-8.68 (m, 2H), 7.91-7.87 (m, 2H), 7.59-7.55 (m, 2H), 7.40-7.33 (m, 2H), 7.22-7.19 (m, 2H), 6.99-6.90 (m, 2H), 4.49 (s, 2H), 3.77 (t, 2H, J= 5.7Hz), 3.63-3.58 (m, 4H), 3.06 (s, 3H), 2.23-2.15 (m, 6H), 1.60-1.41 (m, 2H)

### Beispiel 45: 4-(4-Fluorphenyl)-2-(4-methylsulfinyl)benzylthio-1-N-morpholinoethyl-5-(4-pyridyl)-imidazol

Die im Titel benannte Verbindung wurde nach dem Verfahren aus Beispiel 28 hergestellt, wobei in Schritt (a) 1,3,5-Tri(3-N-morpholinopropyl)hexahydro-1,3,5-triazin und in Schritt (b) zur Benzylierung 4-Methylsulfinyl-benzylchlorid eingesetzt wurde.
IR: 1/λ (cm⁻¹) = 2956, 1601, 1509, 1406, 1220, 1115, 1047, 837, 815
¹H NMR (CDCl₃, ppm): 8.71-8.68 (m, 2H), 7.63-7.50 (m, 4H), 7.41-7.34 (m, 2H), 7.22-7.19 (m, 2H), 6.98-90 (m, 2H), 4.47 (s, 2H), 3.78 (t, 2H, J= 7.8 Hz), 3.60-3:56 (m, 4H), 2.73 (s, 3H), 2.20-2.09 (m, 6H), 1.60-1.42 (m, 2H)

### Beispiel 46: 2-Benzylthio-4-(4-fluorphenyl)-1-(2,2,6,6-tetramethylpiperidin-4-yl)-imidazol

Die im Titel benannte Verbindung wurde nach dem Verfahren aus Beispiel 28 hergestellt, wobei in Schritt (a) 2,2,6,6-Tetramethyl-4-methylenamino-piperidin eingesetzt wurde und zur Benzylierung in Schritt (b) Benzylchlorid.
IR: 1/λ (cm⁻¹) = 2739, 1602, 1510, 1390, 1354, 1224, 1158, 840, 700

### Beispiel 47: 4-(4-Fluorphenyl)-1-methyl-2-N-morpholinoethylthio-5-(4-pyridyl)-imidazol

a) Die Herstellung des 1-substituierten 4-(4-Fluorphenyl)-5-(4-pyridyl)-imidazol-2-thions (Verbindung 5 aus Schema 1) erfolgte wie in Beispiel 1 beschrieben, wobei die zugegebene Iminverbindung 1,3,5-Trimethylhexahydro-1,3,5-triazin war.
b) Anschließend wurden 0,4 g der gewonnenen Thionverbindung (5) zur Substitution des Schwefels unter Schutzgas in 20 mltrockenem Ethanol suspendiert und mit der äquimolaren Menge an N-(2-Chlorethyl)-morpholinhydrochlorid versetzt. Nach Zugabe einer Spatelspitze Na₂CO₃ und einer Spatelspitze Nal wurde das Reaktionsgemisch 5 h unter Rückfluss erhitzt. Nach dem Abkühlen wurden die Salze abfiltriert und das Lösungsmittel am Rotationsverdampfer entfernt. Das Rohprodukt der im Titel benannten Verbindung (Verbindung 10 aus Schema 3) wurde mittels Säulenchromatographie gereinigt.
   Ausbeute:72%
   IR: 1/λ (cm⁻¹) = 2930, 2806, 1602, 1508, 1218, 1131, 1113, 1072, 1007, 865, 850, 829,814
   ¹H NMR (CDCl₃, ppm): 8.70-8.67 (m, 2H), 7.41-7.34 (m, 2H), 7.26-7.21 (m, 2H), 6.97-6.88 (m, 2H), 3.69 (t, 4H, J= 4.6 Hz), 3.50 (s, 3H), 3.39 (t, 3H, J= 6.9 Hz), 2.79 (t, 2H, J= 7.0 Hz), 2.52 (t, 4H, J= 4.6 Hz)

### Beispiel 48: 1-Cis-phenylethenylthio-4-(4-fluorphenyl)-1-methyl-5-(4-pyridyl)-imidazol

a) Die Herstellung des 1-substituierten 4-(4-Fluorphenyl)-5-(4-pyridyl)-imidazol-2-thions (Verbindung 5 aus Schema 1) erfolgte wie in Beispiel 1 beschrieben, wobei die zugegebene Iminverbindung 1,3,5-Trimethylhexahydro-1,3,5-triazin war.
b) Anschließend wurde trockenes Ethanol zu einer Vorlage von 0,1 g metallischem Natrium getropft. 1,2 g der Verbindung (5) aus Schritt (a) wurden zugeben und anschließend mit dem 10-fachen Überschuss an Phenylacetylen versetzt. Das Reaktionsgemisch wurde 6 h unter Rückfluss erhitzt. Nach dem Abkühlen wurde der Ansatz auf Eis gegossen und drei mal mit Petrolether extrahiert. Die organischen Phasen wurden vereinigt. Beim Einengen der organischen Phase am Rotationsverdampfer fiel die im Titel benannte Verbindung (Verbindung 11 aus Schema 3) aus. Ausbeute: 27%
   IR: 1/λ (cm⁻¹) = 3380, 1600, 1513, 1226, 842
   ¹H NMR (CDCl₃, ppm): 8.72-8.69 (m, 2H), 7.52-7.23 (m, 9H), 6.99-6.90 (m, 3H), 6.74 (d, 1H, J= 10.6 Hz), 3.52 (s, 3H)

### Beispiel 49: 1-Cis-phenylethenylthio-4-(4-fluorphenyl)-1-n-propyl-5-(4-pyridyl)-imidazol

Die im Titel genannte Verbindung wurde analog zu Beispiel 48 hergestellt, wobei das in Schritt (a) zugegebene Imin 1,3,5-Tri-n-propylhexahydro-1,3,5-triazin war.
IR: 1/λ (cm⁻¹) = 1596, 1217, 835, 776, 682
¹H NMR (CDCl₃, ppm): 8.74-8.71 (m, 2H), 7.49-7.25 (m, 9H), 7.02-6.89 (m; 3H), 6.73 (d, 1H, J= 10.7 Hz), 3.86 (t, 2H, J= 7.7 Hz), 1.66-1.51 (m, 2H), 0.78 (t, 3H, J= 7.4 Hz)

### Beispiel 50: 2-Cis-phenylethenylthio-1-Cyclopropyl-4-(4-fluorphenyl)-5-(4-pyridyl)-imidazol

Die im Titel genannte Verbindung wurde analog zu Beispiel 48 hergestellt, wobei das in Schritt (a) zugegebene Imin 1,3,5-Tricyclopropylhexahydro-1,3,5-triazin war.
IR: 1/λ (cm⁻¹) = 1596, 1509, 1385, 1217, 832, 785, 686
¹H NMR (CDCl₃, ppm): 8.67-8.64 (m, 2H), 7.54-7.26 (m, 10H), 7.0-6.92 (m, 2H), 6.76 (d, 1H, J= 10.8 Hz), 3.17-3.13 (m, 1H), 1.0-0.96 (m,2H), 0.72-0.68 (m, 2H)

### Beispiel 51: 2-(1,2-Dibrom-2-phenylethyl)thio-4-(4-fluorphenyl)-1-n-propyl-5-(4-pyridyl)-imidazol

Zur Herstellung der im Titel genannten Verbindung wurden 0,4 g des Produkts aus Beispiel 49 (Verbindung 11 aus Schema 3) in CH₂Cl₂ gelöst und die äquimolare Menge an Brom in 15 ml CH₂Cl₂ langsam zugetropft. Das Reaktionsgemisch wurde 5 h bei Raumtemperatur gerührt und anschließend mehrmals mit wässriger Natriumthiosulfatlösung gewaschen. Die organische Phase wurde über Na₂SO₄ getrocknet, das Trocknungsmittel abfiltriert und das Lösungsmittel am Rotationsverdampfer entfernt.
Ausbeute: 98%
IR: 1/λ (cm⁻¹) = 2963, 1631, 1603, 1513, 1224, 1158, 840, 815, 697
¹H NMR (CDCl₃, ppm): 8.74-8.72 (m, 2H), 7.59-7.25 (m, 9H), 6.99-6.90 (m, 2H), 6.21 (d, 1H, J= 7.2 Hz), 5.74 (d, 1H, J= 7.1 Hz), 3.89-3.85 (m, 2H), 1.55-1.51 (m, 2H), 0.77 (t, 3H, J= 7.3 Hz)

### Bisarylthloether

### Beispiel 52: 4-(4-Fluorphenyl)-2-phenylthio-1-n-propyl-5-(4-pyridyl)-imidazol

Es wurde 2-Chlor-1-n-propyl-4-(4-fluorphenyl)-5-(4-pyridyl)-imidazol (Verbindung 7 aus Schema 4) analog zu dem in Beispiel 2 beschriebenen Verfahren hergestellt, wobei das verwendete Amin wie in Beispiel 3B n-Propylamin war.
b) Anschließend wurde die gewonnene Imidazolverbindung (7) zum Bisarylthioether umgesetzt. Dazu wurde NaH (2 Äq.) in 10 ml. trockenem DMF suspendiert und Thiophenol (2 Äq.) langsam zugetropft. Das Reaktionsgemisch wurde 45 min bei Raumtemperatur gerührt und anschießend wurden 0,3 g(4) zugegeben. Der Ansatz wurde 5,5 h unter Rückfluss erhitzt. Nach dem Abkühlen wurde das Gemisch mit destilliertem Wasser versetzt, mit konzentrierter HCl auf einen pH-Wert von 1 eingestellt und sechsmal mit Diethylether gewaschen. Beim Neutralisieren mit 20%iger NaOH fiel die im Titel genannte Verbindung (Verbindung 12 aus Schema 4) aus. Der Niederschlag wurde abfiltriert und über P₂O₅ im Vakuum getrocknet.
   Ausbeute: 70%
   IR: 1/λ (cm⁻¹) = 1509, 1226, 847, 729, 685
   ¹H NMR (CDCl₃, ppm): 8.75-8.72 (m, 2H), 7.45-7.26 (m, 9H), 6.98-6.89 (m, 2H),
   3.95-3.87 (m, 2H), 1.49-1.34 (m, 2H), 0.71 (t, 3H, J= 7.4 Hz)

### Beispiel 53: 2-(4-Chlorphenyl)thio-4-(4-fluorphenyl)-1-n-propyl-5-(4-pyridyl)-imidazol

Es wurde das gleiche Verfahren wie in Beispiel 52 durchgeführt, außer dass in Schritt (b) 4-Chlor-thiophenol verwendet wurde.
IR: 1/λ (cm⁻¹) = 2958, 1601, 1511, 1473, 1226, 1090, 1013, 854, 824
¹H NMR (d₆-DMSO, ppm): 8.74-8.71 (m, 2H), 7.49-7.32 (m, 8H), 7.14-7.05 (m, 2H), 3.92 (t, 2H, J= 7.5 Hz), 1.37-1.26 (m, 2H), 0.59 (t, 3H, J= 7.4 Hz)

### Beispiel 54: 4-(4-Fluorphenyl)-2-(4-methylsulfanyl)phenylsulfanyl-1-n-propyl-5-(4-pyridyl)-imidazol

Es wurde das gleiche Verfahren wie in Beispiel 52 durchgeführt, außer dass in Schritt (b) 4-Methylsulfanyl-thiophenol verwendet wurde.
IR: 1/λ (cm⁻¹) = 2961, 1602, 1510, 1478, 1226, 1103, 851, 796
¹H NMR (CDCl₃, ppm): 8.74-8.71 (m, 2H), 7.43-7.17 (m, 8H), 6.97-6.88 (m, 2H), 3.90 (t, 2H, J= 7.7 Hz), 2.47 (s, 3H), 1.50-1.38 (m, 2H), 0.72 (t, 3H, J= 7.4 Hz)

### Beispiel 55: 4-(4-Fluorphenyl)-2-[(4-methylsulfinyl)phenyl]thio-1-n-propyl-5-(4-pyridyl)-imidazol

0,4 g des Produkts aus Beispiel 54 wurden in 10 ml CH₂Cl₂ gelöst und mit 0,9 Äquivalenten m-Chlorperbenzoesäure versetzt. Das Gemisch wurde 3 h bei Raumtemperatur gerührt. Der entstandene Niederschlag wurde abfiltriert und säulenchromatographisch gereinigt.
Ausbeute: 73%
IR: 1/λ (cm⁻¹) = 1602, 1510, 1226, 1056, 814, 699
¹H NMR (CDCl₃, ppm): 8.77-8.74 (m, 2H), 7.61-7.57 (m 2H), 7.46-7.37 (m, 4H), 7.30-7.28 (m, 2H), 6.98-6.90 (m, 2H), 3.92 (t, 2H, J= 7.7 Hz), 2.72 (s, 3H), 1.49-1.45 (m, 2H), 0.72 (t, 3H, 7.4 Hz)

### Beispiel 56: 4-(4-Fluorphenyl)-2-[(4-methylsulfonyl)phenyl]thio-1-n-propyl-5-(4-pyridyl)-imidazol

0,4 g des Produkts aus Beispiel 54 wurden in 10 ml CH₂Cl₂ gelöst und mit 2,5 Äquivalenten m-Chlorperbenzoesäure versetzt. Das Gemisch wurde 3 h unter Rückfluss erhitzt. Der entstandene Niederschlag wurde abfiltriert und säulenchromatographisch gereinigt.
Ausbeute: 67%
IR: 1/λ (cm⁻¹) = 2967, 1604, 1510, 1316, 1226, 1153, 1094, 1078, 954, 815,771
¹H NMR (CDCl₃, ppm): 8.79-8.76 (m, 2H), 7.88-7.83 (m, 2H), 7.45-7.27 (m, 6H), 6.99-6.90 (m, 2H), 3.92 (t, 2H, J= 7.7 Hz), 3.04 (s, 3H), 1.50- 1.43 (m, 2H), 0.73 (t, 3H, 7.4 Hz)

### 4-Methylimidazolthiole

### Beispiel 57: 1-(4-Fluorphenyl)-4-methyl-5-(4-pyridyl)-imidazol-2-thiol

Es wurde in Analogie zu Beispiel 1 aus dem entsprechenden α-Hydroxyiminoethanon (Verbindung 17 aus Schema 6) die im Titel benannte Verbindung hergestellt.
IR: 1/λ (cm⁻¹) = 3039, 1591, 1516, 1372, 849, 823, 780
¹H NMR (d₆-DMSO, ppm): 12.76 (bs, 1H), 8.47-8.44 (m, 2H), 7.28-7.24 (m, 4H), 7.03-7.0 (m, 2H), 2.19 (s, 3H)

### Beispiel 58: 4-(4-Fluorphenyl)-5-methyl-1-(3-pyridyl)-imidazol-2-thiol

Es wurde in Analogie zu Beispiel 2 aus dem entsprechenden α-Hydroxyiminoethanon (Verbindung 19 aus Schema 6) die im Titel benannte Verbindung hergestellt.
IR: 1/λ (cm⁻¹) = 3035, 1515, 1482, 1430, 1367, 1227, 844, 815, 711
¹H NMR (d₆-DMSO, ppm): 12.67 (bs, 1H), 8.50-8.49 (m, 1H), 8.48-8.47 (m, 1H), 7.69-7.65 (m, 1H), 7.45-7.41 (m, 1H), 7.16-7.12 (m, 4H), 2.08 (s, 3H)

### Regioisomere Thiole

### Beispiel 59: 5-(4-Fluorphenyl)-1-n-propyl-4-(4-pyridyl)-imidazol-2-thiol

a) 1-(4-Fluorphenyl)-2-(4-pyridyl)-α-hydroxyiminoethanon
   Es wurden 2,2 g (0,01 mol) 1-(4-Fluorphenyl)-2-(4-pyridyl)-ethanon (Verbindung 13 aus Schema 5) in 10 ml Eisessig gelöst. Eine Lösung von 0,7 g (0,01 mol) NaNO₂ in 1 ml Wasser wurde langsam zur Vorlage getropft und das Reaktionsgemisch 2 h bei Raumtemperatur gerührt. Es wurden 50 ml destilliertes Wasser zugesetzt und 1 h weitergerührt. Die ausfallende Verbindung, (14), 1-(4-Fluorphenyl)-2-(4-pyridyl)-α-hydroxyiminoethanon, wurde abfiltriert, mit destilliertem Wasser gewaschen und über P₂O₅ im Vakuum getrocknet.
   Ausbeute: 2.0 g (80%)
b) 4-(4-Fluorphenyl)-1-methyl-5-(4-pyridyl)-imidazol-2-thion
   0,5 g der Verbindung (14) wurden zusammen mit der doppelten äquivalenten Menge 1,3,5-Trimethylhexahydro-1,3,5-triazin in 20 ml trockenem Ethanol gelöst und 24 h unter Rückfluss erhitzt. Nach dem Abkühlen wurde das Lösungsmittel am Rotationsverdampfer entfernt. Der Rückstand wurde in 20 ml CHCl₃ aufgenommen, im Eisbad gekühlt und unter Rühren langsam mit 2,2,4,4-Tetramethyl-3-thiono-cyclobutanon versetzt. Das Eisbad wurde entfernt und es wurde 1 h bei Raumtemperatur weitergerührt. Das Lösungsmittel wurde am Rotationsverdampfer entfernt und der erhaltene, ölige Rückstand durch Zugabe von Diethylether verfestigt. Der Niederschlag (15) wurde abfiltriert und durch Umkristallisieren gereinigt.
   Ausbeute: 15%
   IR: 1/λ (cm⁻¹) = 2727, 1604, 1397, 1223, 833, 817
   ¹H NMR (CDCl₃, ppm): 12.03 (bs, 1H), 8.50-8.47 (m, 2H), 7.39-7.20 (m, 4H), 7.10-7.07 (m, 2H), 3.93-3.85 (m, 2H), 1.72-1.56 (m, 2H), 0.82 (t, 3H, J= 7.4 Hz)

### Beispiel 60: 5-(4-Fluorphenyl)-1-N-morpholinoethyl-4-(4-pyridyl)-imidazol-2-thiol

Es wurde wie in Beispiel 59 verfahren, außer dass das Imin in Schritt (b) N-Methylen-(N-morpholino)ethanamin war.
IR: 1/λ (cm⁻¹) = 2803, 1605, 1220, 1118, 849, 833
¹H NMR (d₆-DMSO): 13.09 (bs, 1H), 8.44-8.41 (m, 2H), 7.63-7.56 (m, 2H), 7.47-7.38 (m, 2H), 7.14-7.11 (m, 2H), 3.93 (t, 2H, J= 6.8 Hz), 3.44 (t, 4H, J= 4.5 Hz), 2.42 (t, 2H, J= 6.8 Hz), 2.14 (t, 4H, J= 4.4 Hz)

### Beispiele 61 bis 65

### (Die Nummern der Verbindungen beziehen sich auf Schema 8)

a) 2-Acetamido-4-methylpyridin (25).
   200.0 g 2-Aminopicolin werden mit 400 ml Acetanhydrid und mit 100 mg 4-Dimethylaminopyridin versetzt und 5h refluxiert. Nach dem Abkühlen wird das überschüssige Acetanydrid weitgehend abdestilliert, der Rückstand auf Eis gegossen und mit wässriger Ammoniaklösung neutralisiert. Der dabei ausfallende Niederschlag (25) wird abfiltriert und im Vakuum über P₂O₅ getrocknet.
   Ausbeute: 209.0 g (75 %)
b) 2-Acetamidopyridin-4-carbonsäure (26)
   214.0 g (25) werden unter Rühren in eine 50°C warme wässrige Lösung von 160 g Kaliumpermanganat portionsweise eingetragen. Weitere 360 g Kaliumpermanganat werden innerhalb einer Stunde portionsweise zugesetzt. Dabei sollte die Temperatur des Reaktionsgemisches 90°C nicht übersteigen. Die Mischung wird noch 1,5 h nachgerührt, heiß filtriert und das Filtrat mit konz. HCl auf pH 3-4 eingestellt. Der dabei ausfallende weiße Niederschlag (26) wird abfiltriert und im Vakuum über P₂O₅ getrocknet.
   Ausbeute: 108.0 g (42 %)
c) 2-Cyano-2-(4-fluorphenyl)-1-(2-acetamido-4-pyridyl)ethanon (27)
   18.0 g (26) werden in 50 ml Dimethylformamid (DMF) abs. aufgenommen, mit 17.0 g Carbonyldiimidazol (CDI) versetzt und 45 min. bei Raumtemperatur gerührt. Anschließend werden 14.9 g 4-Fluoracetonitril und 14.6 g Kalium-tert-butanolat zugegeben und das Reaktionsgemisch wird 2 h auf 120 °C erhitzt. Nach dem Abkühlen wird der Ansatz über Nacht bei Raumtemperatur gerührt. Die Lösung wird dann mit Eis versetzt und mit konz. HCl neutralisiert. Der dabei ausfallende Niederschlag (27) wird abfiltriert und im Vakuum über P₂O₅ getrocknet.
   Ausbeute: 18.1 g (65%)
d) 2-(4-Fluorphenyl)-1-(2-amino-4-pyridyl)ethanon (28)
   27.9 g (27) werden mit 150 ml 48 %iger Bromwasserstoffsäure versetzt und das Reaktionsgemisch 30 h unter schwachem Sieden gehalten. Nach dem Abkühlen wird der Ansatz auf Eis gegossen und mit konzentriertem Ammoniak neutralisiert. Der dabei ausfallende Niederschlag (28) wird scharf abgesaugt, mehrmals mit Petrolether und kaltem Diethylether gewaschen und getrocknet.
   Ausbeute: 11.7 g (55 %)
e) 2-(4-Fluorphenyl)-1-(2-acetamido-4-pyridyl)ethanon (29)
   12.0 g der Verbindung (28) werden in 100 ml Acetanhydrid suspendiert, mit einer Spatelspitze 4- Dimethylaminopyridin versetzt und das Reaktionsgemisch wird 5 h refluxiert. Das überschüssige Acetanhydrid wird weitgehend abdestilliert, der Rückstand hydrolysiert und mit konz. Ammoniak auf pH 7 eingestellt. Der dabei ausfallende, helle Niederschlag (29) wird abfiltriert und im Vakuum über P₂O₅ getrocknet.
   Ausbeute: 13.5 g (94%)
f) 2-(4-Fluorphenyl)-1-(2-acetamido-4-pyridyl)-α-hydroxyiminoethanon (3)
   2.1 g Natriummethylatlösung (30% in Methanol) werden mit 30 ml Methanol versetzt und zu einer Lösung von 1.2 g Isoamylnitrit in 20 ml Methanol gegeben. Unter Rühren werden portionsweise 3.0 g (29) zugegeben und anschließend wird 2 h bei Raumtemperatur weitergerührt. Das Lösungsmittel wird abdestilliert, der feste Rückstand in Wasser aufgenommen und mit 10 %iger HCl auf pH 7 eingestellt. Der dabei ausfallende, helle Niederschlag (30) wird abfiltriert und im Vakuum über P₂O₅ getrocknet.
   Ausbeute: 1.8 g (54 %)
g) Herstellung der Verbindungen (31):
   (30) wird zusammen mit der doppelten Menge des entsprechenden Triazins in absolutem Ethanol gelöst und solange refluxiert, bis sich das Edukt vollständig umgesetzt hat. Nach dem Abkühlen wird Ethanol am Rotationsverdampfer entfernt. Der teilweise ölige Rückstand verfestigt sich bei Zugabe von Diethylether. Der Niederschlag an Verbindungen 31 wird abfiltriert und im Vakuum getrocknet.
   Ausbeuten:
   R1= -CH₃: 74 %
   R1= -C₃H₇: 62 %
   R1= 2,2,6,6-Tetramethylpiperidin-4-yl: 81 %
   R1= N-Morpholinopropyl-: 72 %
   R1= 3-Hydroxypropyl-: 56 %
h) Herstellung der Verbindungen (32)
   Verbindung (31) wird in CHCl₃ gelöst und das Reaktionsgemisch im Eisbad abgekühlt Eine äquimolare Lösung von 2,2,4,4-Tetramethylcyclobutan-3-thioxobutanön in CHCl₃ wird langsam zur Vorlage getropft und anschließend 30 min. im Eisbad gerührt. Das Eisbad wird entfernt und 1 h bei Raumtemperatur weitergerührt. Anschließend wird das Lösungsmittel am Rotationverdampfer entfernt und der feste Rückstand in Diethylether ausgerührt. Der Niederschlag (32) wird abfiltriert und im Vakuum getrocknet.
   Ausbeuten:
   R1= -CH₃: 96 %
   R1= -C₃H₇: 74 %
   R1=2,2,6,6-Tetramethylpiperidin-4-yl: 61%
   R1= N-Morpholinopropyl-: 82 %
   R1= 3-Hydroxypropyl-: 71 %
i) Herstellung der Verbindungen (33)
   Verbindung (32) wird unter Schutzgas in abs. Ethanol suspendiert und mit der äquimolaren Menge an Methyliodid versetzt. Nach Zugabe einer Spatelspitze Na₂CO₃ wird das Reaktionsgemisch solange refluxiert, bis sich das Edukt vollständig umgesetzt hat. Nach dem Abkühlen werden die anorganischen Salze abfiltriert und das Lösungsmittel am Rotationsverdampfer entfernt. Das Rohprodukt (33) wird mittels Säulenchromatographie gereinigt.

### Beispiel 61: 4-(4-Fluorphenyl)-1-methyl-5-(2-acetamido-4-pyridyl)-2-methylthioimidazol

R1= -CH₃ : Ausbeute 63 %
NMR (CDCl₃, ppm): 8.75 (bs, 1H), 8.26-8.24 (m, 2H), 7.46- 7.39 (m, 2H), 6.97- 6.88 (m, 3H), 3.53 (s, 3H), 2.71 (s, 3H), 2.23 (s, 3H)
IR (1/cm): 1669, 1607, 1543, 1505, 1416, 1268, 1218, 843

### Beispiel 62: 4-(4-Fluorphenyl)-1-n-propyl-5-(2-acetamido-4-pyridyl)-2-methylthioimidazol

R1= -C₃H₇: Ausbeute 28 %
NMR (CDCl₃, ppm): 8.28- 8.25 (m, 2H), 7.44-7.37 (m, 2H), 6.96-6.88 (m, 2H), 3.85 (t, 2H, J= 7.7 Hz), 2.73 (s, 3H), 2.24 (s, 3H), 1.65-1.57 (m, 2H), 0.83 (t, 3H, J=7.4 Hz) IR (1/cm): 3303, 1674, 1544, 1501, 1416, 1264, 1213, 845

### Beispiel 63: 4-(4-Fluorphenyl)-1-(2,2,6,6-tetramethylpiperidin-4-yl)-5-(2-acetamido-4-pyridyl)-2-methylthioimidazol

R1=2,2,6,6-Tetramethylpiperidin-4-yl: Ausbeute 23 %
NMR (CDCl₃, ppm): 10.62 (s, 1H), 8.38-8.35 (m, 2H), 8.01 (s, 1H), 7.33-7.26 (m, 2H), 7.04-6.95 (m, 3H), 4.19- 4.03 (m, 1H), 261 (s, 3H); 2.00 (s, 3H), 1.87 -1.81 (m, 2H), 1.52-1.47 (m, 2H), 0.93 (s, 6H), 0.78 (s, 6H)
IR (1/cm): 2976, 1699, 1533, 1407, 1255, 838

### Beispiel 64: 4-(4-Fluorphenyl)-1-[3-(N-morpholino)-propyl]-5-(2-acetamido-4-pyridyl)-2-methylthioimidazol

R1= N-Morpholinopropyl-: Ausbeute 52 %
NMR (CDCl₃, ppm):8.29 (m, 1H), 8.12 (s, 1H), 7.42-7.35 (m, 2H), 6.96-6.87 (m, 3H), 4.08-3.92 (m, 6H), 3.17-3.00 (m, 6H), 2.74 (s, 3H), 2.41-2.34 (m, 2H), 2.24 (s, 3H)

### Beispiel 65: 4-(4-Fluorphenyl)-1-(3-hydroxypropyl)-5-(2-acetamido-4-pyridyl)-2-methylthioimidazol

R1= 3-Hydroxypropyl-: Ausbeute 32 %
NMR(CDCl₃, ppm):8.69 (bs, 1H), 8.23-8.19 (m, 2H), 7.44-7.37 (m, 2H), 6.98-6.86 (m, 3H), 4.04 (t, 2H, J= 7.9 Hz), 3.70 (t, 2H, J= 7.2 Hz), 2.74 (s, 3H), 2.25 (s, 3H), 2.13-2.05 (m, 2H)

### Beispiel 66: 4-(4-Fluorphenyl)-1-methyl-5-(2-amino-4-pyridyl)-2-methylthioimidazol

Die Verbindung des Beispiels 61 wird in 10 %iger HCl gelöst und 14 h refluxiert. Nach dem Abkühlen wird mit 20 %iger NaOH neutralisiert. Der dabei ausfallende, helle Niederschlag wird abfiltriert und im Vakuum über P₂O₅ getrocknet.
Ausbeute: 82 %
NMR (CDCl₃, ppm): 8.16-8.13 (m, 1H), 7.50-7.43 (m, 2H), 6.98-6.89 (m, 2H), 6.60-6.57 (m, 1H), 6.41 (s 1H), 4.60 (bs, 2H,) 3.46 (s, 3H), 2.70 (s, 3H)
IR (1/cm): 1629, 1542, 1509, 1215, 837, 814

### Beispiele 67 bis 69

Die Verbindung des Beispiels 66 wird in abs. Tetrahydrofuran (THF) gelöst und mit der 1.2-fachen Menge an Triethylamin versetzt. Das Reaktionsgemisch wird im Eisbad abgekühlt. Unter Rühren wird die 1.2-fache Menge des Säurechlorids zugetropft und solange weitergerührt, bis kein Edukt mehr vorhanden ist. Das Reaktionsgemisch wird filtriert und das Filtrat zur Trockne eingeengt. Die Reinigung des Rohprodukts erfolgt durch Säulenchromatographie.

### Beispiel 67: 4-(-Fluorphenyl)-1-methyl-5-[2-(4-methoxybenzamido)-4-pyridyl]-2-methylthioimidazol

Ausbeute: 62%
NMR (CDCl₃, ppm): 8.66 (s, 1H), 8.44 (s, 1H), 8.30 (s, 1H), 8.29-8.28 (m, 1H), 7.94-7.89 (m, 2H), 7.49-7.42 (m, 2H), 6.95- 6.90 (m, 4H), 3. 90 (s, 3H), 3.58 (s, 3H), 2.72 (s, 3H)
IR (1/cm): 3410, 1674, 1500, 1412, 1253, 1175, 840, 759

### Beispiel 68: 4-(-Fluorphenyl)-1-methyl-5-(2-cyclopropylamido-4-pyridyl)-2-methylthioimidazol

Ausbeute: 24%
NMR (CDCl₃, ppm): 8.67-8.62 (m, 1H), 7.63-7.38 (m, 3H), 6.98-6.85 (m, 3H), 3.90 (s, 3H), 2.73 (s, 3H), 2.05-1.98 (m, 1H), 1.26-1.14 (m, 2H), 1.21-1.14 (m, 2H)

### Beispiel 69: 4-(-Fluorphenyl)-1-methyl-5-(2-cyclopentylamido-4-pyridyl)-2-methylthioimidazol

Ausbeute: 53%
NMR (CDCl₃, ppm): 8.28-8.22 (m, 3H), 7.46-7.39 (m, 2H), 6.97-6.87 (m, 3H), 3.54 (s, 3H), 2.69 (s, 3H), 1.97-1.67 (m, 8H)

### Beispiele 70 bis 72

1.2 eq NaH werden in DMF suspendiert, die Verbindung des Beispiels 66 wird langsam zugegeben und das Reaktionsgemisch noch 1 h bei Raumtemperatur gerührt. Danach wird das Benzylbromid bzw. Phenylethylbromid äquimolar zugegeben und solange refluxiert, bis kein Edukt mehr vorhanden ist. Man verdünnt das Reaktionsgemisch mit Wasser und filtriert den dabei ausfallenden Niederschlag ab. Die Reinigung des Rohprodukts erfolgt durch Säulenchromatographie.

### Beispiel 70: 4-(4-Fluorphenyl)-1-methyl-5-(2-benzylamino-4-pyridyl)-2-methylthioimidazol

Ausbeute: 13 %
NMR (CDCl₃, ppm): 8.12-8.16 (m, 1H), 7.47-7.26 (m, 7H), 6.95- 6.86 (m, 2H); 6.53-6.50 (m, 1H), 6.24 (s, 1H), 5.30 (bs, 1H), 4.47 (d, 2H, J= 5.8 Hz), 3.32 (s, 3H), 2.68 (s, 3H)
IR(1/cm): 3241, 1610, 1507, 1219, 839, 813, 737, 698

### Beispiel 71: 4-(4-Fluorphenyl)-1-methyl-5-[2-(2-phenylethyl)amino-4-pyridyl]-2-methyl-thioimidazol

Ausbeute: 54 %
NMR (CDCl₃, ppm): 8.12-8.10 (m, 1H), 7.41- 7.19 (m, 7H), 6.92- 6.84 (m, 2H), 6.46-6.43 (m, 1H), 6.06 (s, 1H), 5.18 (d, 1H, J= 6.3 Hz), 4.63-4.57 (m, 1H), 3.11 (s, 3H), 2.70 (s, 3H),
IR (1/cm): 1605, 1505, 1432, 1219, 839, 701

Bei Zusatz des Benzylbromids in 2.5-facher Menge erfolgt Bisubstitution des Stickstoffs (13).

### Beispiel 72: 4-(4-Fluorphenyl)-1-methyl-5-(2-dibenzylamino-4-pyridyl)-2-methylthioimidazol

Ausbeute: 81%
NMR (CDCl₃, ppm): 8.27-8.24 (m, 1H), 7.45- 7.19 (m, 12 H), 6.95- 6.86 (m, 2H), 6.51-6.48 (m, 1H), 6.31 (s, 1H), 4.80 (s, 4H), 3.17 (s, 3H), 2.66 (s, 3H)
IR (1/cm): 1598, 1496, 1427, 1219, 840, 831, 734, 702

### Beispiele 73 bis 75

Die Verbindung der Beispiele 61, 62 bzw. 63 wird in THF gelöst und unter Rühren mit dem 10-fachen Überschuss an LiAlH₄ versetzt. Anschließend wird das Reaktionsgemisch 2 h erhitzt. Nach dem Abkühlen wird Wasser langsam zugegeben. Das Gemisch wird mehrmals mit CH₂Cl₂ extrahiert und die vereinigten organischen Phasen werden über Na₂SO₄ getrocknet. Das Trocknungsmittel wird abfiltriert und das Lösungsmittel entfernt. Das Rohprodukt wird mittels Säulenchromatographie gereinigt.

### Beispiel 73: 4-(4-Fluorphenyl)-1-methyl-5-(2-ethylamino-4-pyridyl)-2-methylthioimidazol

Ausbeute: 70%
NMR (CDCl₃, ppm): 8.17-8.15 (m, 1H), 7.53-7.46 (m, 2H), 6.98-6.89 (m, 2H), 6.52-6.49 (m, 1H), 6.27-6.26 (m, 1H), 4.59 (t, 1H, J= 6.0 Hz), 3.47 (s, 3H), 3.29-3.23 (m, 2H), 2.70 (s, 3H), 1.23 (t, 3H, J= 7.1 Hz)
IR (1/cm): 3235, 1604, 1562, 1506, 1435, 1221, 844, 806

### Beispiel 74: 4-(4-Fluorphenyl)-1-n-propyl-5-(2-ethylamino-4-pyridyl)-2-methylthioimidazol

Ausbeute: 25 %
NMR (CDCl₃, ppm): 8.17-8.14 (m, 1H), 7.51-7.43 (m, 2H), 6.98-6.87 (m, 2H), 6.53-6.50 (m, 1H), 6.27 (s, 1H), 4.61 (t, 1H, J= 2. 8 Hz), 3.79 (t, 2H, 7.7 Hz), 3.28- 3.22 (m, 2H), 2.71 (s, 3H), 1.66- 1.54 (m, 2H), 1.24 (t, 3H, J= 7.2 Hz), 0.83 (t, 3H, J= 7.4 Hz)
IR (1/cm): 3275, 2930, 1607, 1525, 1507, 1219, 846, 813

### Beispiel 75: 4-(4-Fluorphenyl)-1-(2,2,6,6-tetramethylpiperidin-4-yl)-5-(2-ethylamino-4-pyridyl)-2-methylthioimidazol

Ausbeute: 52 %
NMR (CDCl₃, ppm): 8.10- 8.07 (m, 2H), 7.47- 7.40 (m, 2H), 7.12-7.03 (m, 2H), 6.44-6.41 (m, 1H), 6.37 (s, 1H), 4.30-4.14 (m, 1H), 3.27- 3.21 (m, 2H), 2.66 (s, 3H), 2.11-1.91 (m, 2H), 1.59-1.52 (m, 2H), 1.12-1.01 (m, 9H), 0.90 (s, 6H)
IR (1/cm):3325, 2959, 1603, 1516, 1499, 1217, 1158, 849, 812

### Beispiel 76: 4-(4-Fluorphenyl)-1-(3-N-morpholinopropyl)-5-(2-acetamido-4-pyridyl)-2-(4-methylsulfinylbenzyl)thioimidazol

4-(4-Fluorphenyl)-1-(3-N-morpholinopropyl)-5-(2-acetamido-4-pyridyl)-imidazol-2-thion wird unter Schutzgas in abs. Ethanol suspendiert und mit der äquimolaren Menge des 4-Methylsulfinylbenzylchlorids versetzt. Nach Zugabe einer Spatelspitze Na₂CO₃ wird das Reaktionsgemisch solange refluxiert, bis sich das Edukt vollständig umgesetzt hat. Nach dem Abkühlen werden die anorganischen Salze abfiltriert und das Lösungsmittel am Rotationsverdampfer entfernt. Das Rohprodukt wird mittels Säulenchromatographie gereinigt.
Ausbeute: 27 %
NMR (CDCl₃, ppm): 8.67 (bs, 1H), 8.28-8.25 (m, 2H), 8.12 (s, 1H), 7.64-7-49 (m, 4H), 7.44-7.37 (m, 2H), 6.98-6.86 (m, 3H), 4.45 (s, 2H), 3.81-3.65 (m, 6H), 2.72 (s, 3H), 2.54-2.52 (m, 6H), 2.22 (s, 3H), 1.85-1.73 (m, 2H)

Nach dem oben beschriebenen Verfahren wurden folgende Verbindungen erhalten:

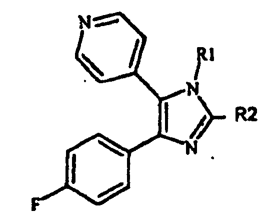

| Beispiel | R¹ | R² |
|---|---|---|
| 77 | Cyclo-C₆H₁₁ | CH₃ |
| 78 | -(CH₂)₂-N(CH₃)₂ | CH₃ |
| 79 | -(CH₂)₃-OH | CH₃ |
| 80 | -(CH₂)₃-OCH₃ | CH₃ |
| 81 | CH₃ | -(CH₂)₂ S CH₃ |
| 82 | CH₃ | -(CH₂)₂ SO CH₃ |
| 83 | Cyclo-C₆H₁₁ | CH₂-Ph-4-SO CH₃ |
| 84 | Cyclo-C₆H₁₁ | CH₂-Ph-4-SCH₃ |

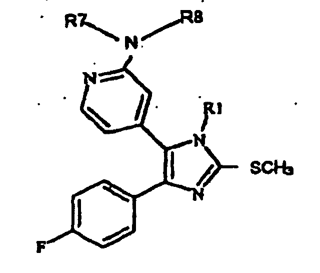

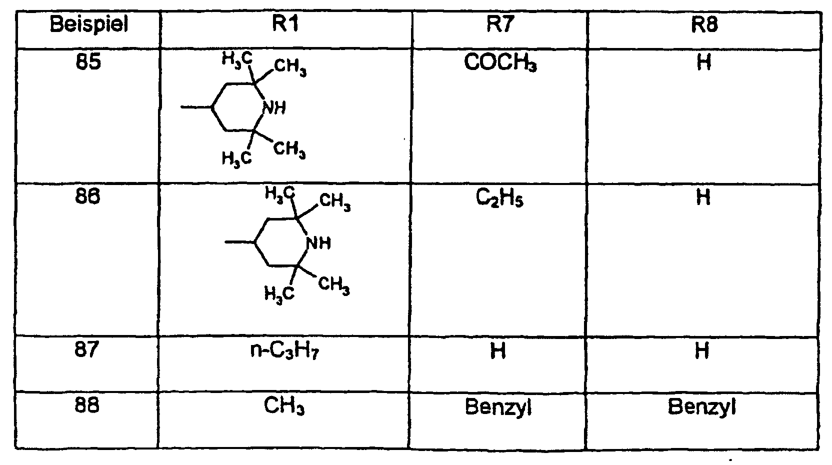

## Patentansprüche

1. 2-Thio-substituierte Imidazolverbindungen der Formel I 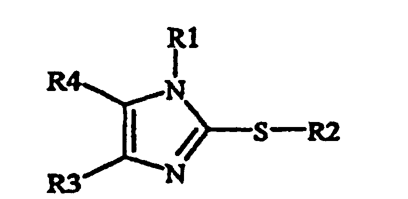 worin
R¹ ausgewählt ist unter:
C₁-C₆-Alkyl, das gegebenenfalls durch eine oder zwei Hydroxy- oder C₁-C₄-Alkoxygruppen, oder einen nicht-aromalischen heterocyclischen Rest mit 5 oder 6 Ringatomen und 1 oder 2 Heteroatomen, die unabhängig voneinander ausgewählt sind unter N, O und S, substituiert ist,
C₂-C₆-Alkenyl,
C₃-C₆-Cycloalkyl,
Aryl, das gegebenenfalls durch ein oder mehrere Halogenatome oder eine C₁-C₄-Alkylsulfanylgruppe substituiert ist,
Amino-C₁-C₄-alkyl, wobei die Aminogruppe gegebenenfalls durch ein oder zwei C₁-C₄-Alkylgruppen substituiert ist,
Aminoaryl, wobei die Aminogruppe gegebenenfalls durch ein oder zwei C₁-C₄-Alkylgruppen substituiert ist,
Aryl-C₁-C₄₋alkyl oder
einem aromatischen oder nicht-aromatischen heterocyclischen Rest mit 5 oder 6 Ringatomen und 1 oder 2 Heteroatomen, die unabhängig voneinander ausgewählt sind unter N, O und S, der gegebenenfalls durch 1, 2, 3 oder 4 C₁-C₄-Alkylgruppen, eine Aryl- oder Aryl-C₁-C₄-alkylgruppe substituiert ist,
R² ausgewählt ist unter
C₁-C₆-Alkyl,
Phenyl-C₁-C₄-alkyl, wobei die Phenylgruppe einen oder zwei Substituenten aufweisen kann, die unabhängig voneinander unter C₁-C₄-Alkyl, Halogen, C₁-C₄-Alkylsulfanyl, C₁-C₄-Alkylsulfinyl und C₁-C₄-Alkylsulfonyl ausgewählt sind,
C₂-C₆-Alkenyl,
C₂-C₆-Alkenyl, das durch ein oder zwei Halogenatome und/oder Phenylgruppen substituiert ist, wobei die Phenylgruppe unabhängig durch ein oder zwei C₁-C₄-Alkyl oder Halogenatome substituiert sein kann,
C₂-C₆-Alkinyl,
C₂-C₆-Alkinyl, das durch eine Phenylgruppe substituiert ist, die gegebenenfalls durch ein oder zwei C₁-C₄-Alkyl oder Halogenatome substituiert sein kann,
C₁-C₆-Alkyl, das durch einen nicht-aromatischen heterocyclischen Rest mit 5 oder 6 Ringatomen und 1 oder 2 Heteroatomen, die unabhängig voneinander ausgewählt sind unter N, O, und S, C₁-C₄-Alkylsulfanyl, C₁-C₄-Alkylsulfinyl oder C₁-C₄-Alkylsulfonyl substituiert ist,
Phenyl oder
Phenyl steht, das einen oder zwei Substituenten aufweist, die unabhängig voneinander unter C₁-C₄-Alkyl, Halogen, C₁-C₄-Alkylsulfanyl, C₁-C₄-Alkylsulfinyl oder C₁-C₄-Alkylsulfonyl ausgewählt sind, oder
R¹ und R² zusammen für -CH₂CH₂- oder -CH₂CH₂CH₂- stehen,
R³ für halogensubstituiertes Phenyl steht,
R⁴ für 4-Pyridyl steht, das substituiert ist durch Amino, C₁-C₄-Alkylamino, Phenyl-C₁-C₄-alkylamino oder R⁵CONR⁶-, worin R⁵ für C₁-C₄-Alkyl, phenyl, das einen oder zwei Substituenten aufweisen kann, die unabhängig voneinander unter C₁-C₄-Alkyl, C₁-C₄-Alkoxy und Halogen ausgewählt sind, oder C₃-C₆-Cycloalkyl steht und R⁶ für H, C₁-C₄-Alkyl oder Benzyl steht,
mit der Einschränkung, dass wenn R¹ für Aryl- C₁-C₅-alkyl oder Amino--C₁-C₆-alkyl steht, wobei die Aminogruppe gegebenenfalls durch eine oder zwei C₁-C₄-Alkylgruppen substituiert ist, R² für Alkylsulfonyl- oder Alkylsulfinylaryl-C₁-C₅-alkyl steht,
und die optischen Isomere und physiologisch verträglichen Salze davon.

2. Verbindungen nach Anspruch 1, wobei der Pyridylrest in 2-Position substituiert ist.

3. Verbindungen nach Anspruch 1 oder 2, wobei R⁴ für 4-Pyridyl steht, das substituiert ist durch Phenyl-C₁-C₄-alkylamino.

4. Verbindungen nach einem der vorhergehenden Ansprüche, wobei R³ für 4-Fluorphenyl steht.

5. Verbindungen nach einem der vorhergehenden Ansprüche, wobei R1 für C₁-C₃-Alkyl, C₃-C₆-Cycloalkyl oder 2,2,6,6-Tetramethylpiperidinyl steht.

6. Verbindungen nach Anspruch 5, wobei R¹ für C₁-C₃-Alkyl oder 2,2,6,6-Tetramethylpiperidinyl steht.

7. Verbindungen nach einem der vorhergehenden Ansprüche, wobei R2 für C₁-C₆-Alkyl oder Phenyl-C₁-C₄-alkyl steht, wobei die Phenylgruppe durch C₁-C₄-Alkylsulfanyl, C₁-C₄-Alkylsulfinyl oder C₁-C₄-Alkylsulfonyl substituiert ist.

8. Verbindungen der Formel I, worin R¹ für C₁-C₆-Alkyl, das gegebenenfalls durch eine oder zwei Hydroxy- oder C₁-C₄-Alkoxygnrppen, oder einen nicht-aromatischen heterocyclischen Rest mit 5 oder 6 Ringatomen und 1 oder 2 Heteroatomen, die unabhängig voneinander ausgewählt sind unter N, O und S, substituiert ist, C₃-C₆-Cycloalkyl oder einen aromatischen oder nicht-aromatischen heterocyclischen Rest mit 5 oder 6 Ringatomen und 1 oder 2 Heteroatomen, die unabhängig voneinander ausgewählt sind unter N, O und S, der gegebenenfalls durch 1, 2, 3 oder 4 C₁-C₄-Alkylgruppen, eine Aryl- oder Aryl-C₁-C₄-alkylgruppe substituiert ist, steht, R2 für C₁-C₆-Alkyl, Phenyl-C₁-C₄-alkyl, wobei die Phenylgruppe einen oder zwei Substituenten aufweisen kann, die unabhängig voneinander unter C₁-C₄-Alkyl, Halogen, C₁-C₄-Alkylsulfanyl, C₁-C₄-Alkylsulfinyl und C₁-C₄-Alkylsulfonyl ausgewählt sind, Phenyl, C₁-C₆-Alkyl, das durch einen nicht-aromatischen heterocyclischen Rest mit 5 oder 6 Ringatomen und 1 oder 2 Heteroatomen, die unabhängig voneinander ausgewählt sind, unter N, O und S, C₁-C₄-Alkylsulfanyl, C₁-C₄-Alkylsulfinyl oder C₁-C₄-Alkylsulfonyl substituiert ist, oder Phenyl steht, das einen oder zwei Substituenten aufweist, die unabhängig voneinander unter C₁-C₄-Alkyl, Halogen, C₁-C₄-Alkylsulfanyl, C₁-C₄-Alkylsulfinyl oder C₁-C₄-Alkylsulfonyl ausgewählt sind,
R¹ und R² zusammen für -CH₂CH₂- oder -CH₂CH₂CH₂- stehen, R³ für halogensubstituiertes Phenyl steht, R⁴ für 4-Pyridyl steht, das substituiert ist durch Amino, C₁-C₄-Alkylamino, Phenyl-C₁-C₄-alkylamino oder R⁵CONR⁶-, worin R⁵ für C₁-C₄-Alkyl, Phenyl, das einen oder zwei Substituenten aufweisen kann, die unabhängig voneinander unter C₁-C₄-Alkyl, C₁-C₄-Alkoxy und Halogen ausgewählt sind, oder C₃-C₆-Cycloalkyl steht und R⁶ für H, C₁-C₄-Alkyl oder Benzyl steht,
und die optischen Isomere und physiologisch verträglichen Salze davon.

9. Verbindungen nach Anspruch 8, wobei R1 die in einem der Ansprüche 5 oder 6 angegebenen Bedeutungen besitzt.

10. Verbindungen nach Anspruch 8 oder 9, wobei R² die in Anspruch 7 angegebenen Bedeutungen besitzt.

11. Verbindungen nach einem der Ansprüche 8 bis 10, wobei R⁴ die in Anspruch 3 angegebenen Bedeutungen besitzt.

12. Pharmazeutische Mittel, enthaltend wenigstens eine Verbindung nach einem der Ansprüche 1 bis 11, gegebenenfalls zusammen mit ein oder mehreren pharmazeutisch akzeptablen Träger- und/oder Zusatzstoffen.

13. Verwendung wenigstens einer der Verbindungen nach einem der Ansprüche 1 bis 11 zur Herstellung eines pharmazeutischen Mittels zur Behandlung von Erkrankungen, die mit einer Störung des Immunsystems im Zusammenhang stehen.

## Claims

1. A 2-thio-substituted imidazole derivative of the formula I 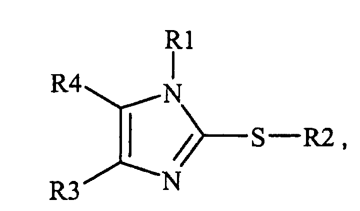 wherein
R¹ is selected from:
C₁-C₆-alkyl which is unsubstituted or substituted by one or two hydroxyl or C₁-C₄-alkoxy groups or by a nonaromatic heterocyclic radical having 5 or 6 ring atoms and 1 or 2 heteroatoms independently of one another selected from N, O and S,
C₂-C₆-alkenyl,
C₃-C₆-cycloalkyl,
aryl which is unsubstituted or substituted by one or more halogen atoms or by a C₁-C₄-alkylsulfanyl group,
amino-C₁-C₄-alkyl, where the amino group is unsubstituted or substituted by one or two C₁-C₄-alkyl groups,
aminoaryl, where the amino group is unsubstituted or substituted by one or two C₁-C₄-alkyl groups,
aryl-C₁-C₄-alkyl or
an aromatic or nonaromatic heterocyclic radical having 5 or 6 ring atoms and 1 or 2 heteroatoms independently of one another selected from N, O and S, which heterocyclic radical is unsubstituted or substituted by 1, 2, 3 or 4 C₁-C₄-alkyl groups, an aryl or aryl-C₁-C₄-alkyl group,
R² is selected from:
C₁-C₆-alkyl,
phenyl-C₁-C₄-alkyl, where the phenyl group may have one or two substituents independently of one another selected from C₁-C₄-alkyl, halogen, C₁-C₄-alkylsulfanyl, C₁-C₄-alkylsulfinyl and C₁-C₄-alkylsulfonyl,
C₂-C₆-alkenyl,
C₂-C₆-alkenyl which is substituted by one or two halogen atoms and/or phenyl groups, where the phenyl group may independently be substituted by one or two C₁-C₄-alkyl or halogen atoms,
C₂-C₆-alkynyl,
C₂-C₆-alkynyl which is substituted by a phenyl group which may be unsubstituted or substituted by one or two C₁-C₄-alkyl or halogen atoms,
C₁-C₆-alkyl which is substituted by a nonaromatic heterocyclic radical having 5 or 6 ring atoms and 1 or 2 heteroatoms independently of one another selected from N, O and S, C₁-C₄-alkylsulfanyl, C₁-C₄-alkylsulfinyl or C₁-C₄-alkylsulfonyl,
phenyl or
phenyl which has one or two substituents independently of one another selected from C₁-C₄-alkyl, halogen, C₁-C₄-alkylsulfanyl, C₁-C₄-alkylsulfinyl and C₁-C₄-alkylsulfonyl, or
R¹ and R² together are -CH₂CH₂- or -CH₂CH₂CH₂-,
R³ is halogen substituted phenyl,
R⁴ is 4-pyridyl which is substituted by amino, C₁-C₄-alkylamino, phenyl-C₁-C₄-alkylamino or R⁵CONR⁶-, where R⁵ is C₁-C₄-alkyl, phenyl, which may have one or two substituents independently of one another selected from C₁-C₄-alkyl, C₁-C₄-alkoxy and halogen, or C₃-C₆-cycloalkyl and R⁶ is H, C₁-C₄-alkyl or benzyl, and
with the proviso that, if R¹ represents aryl-C₁-C₅-alkyl or amino-C₁-C₆-alkyl, where the amino group is unsubstituted or substituted by one or two C₁-C₄-alkyl groups, R² represents alkylsulfonyl- or alkylsulfinylaryl-C₁-C₅-alkyl,
and the optical isomers and physiologically acceptable salts thereof.

2. A compound as claimed in claim 1, wherein the pyridyl is substituted in 2-position.

3. A compound as claimed in claim 1 or 2, wherein R⁴ is 4-pyridyl which is substituted by phenyl-C₁-C₄-alkylamino.

4. A compound as claimed in any of the preceding claims, wherein R³ is 4-fluorophenyl.

5. A compound of the formula I as claimed in any of the preceding claims, wherein R¹ is C₁-C₃-alkyl, C₃-C₆-cycloalkyl or 2,2,6,6-tetramethylpiperidinyl.

6. A compound as claimed in claim 5, wherein R¹ is C₁-C₃-alkyl or 2,2,6,6-tetramethylpiperidinyl.

7. A compound of the formula I as claimed in any of the preceding claims, wherein R² is C₁-C₆-alkyl or phenyl-C₁-C₄-alkyl, where the phenyl group is substituted by C₁-C₄-alkylsulfanyl, C₁-C₄-alkylsulfinyl or C₁-C₄-alkylsulfonyl.

8. A compound of the formula I, wherein R¹ is C₁-C₆-alkyl which is unsubstituted or substituted by one or two hydroxyl or C₁-C₄-alkoxy groups, or a nonaromatic heterocyclic radical having 5 or 6 ring atoms and 1 or 2 heteroatoms independently of one another selected from N, O and S, C₃-C₆-cycloalkyl or an aromatic or nonaromatic heterocyclic radical having 5 or 6 ring atoms and 1 or 2 heteroatoms independently of one another selected from N, O and S, which heterocyclic radical is unsubstituted or substituted by 1, 2, 3 or 4 C₁-C₄-alkyl groups, an aryl or aryl-C₁-C₄-alkyl group,
R² is, C₁-C₆-alkyl, phenyl-C₁-C₄-alkyl, where the phenyl group may have one or two substituents independently of one another selected from C₁-C₄-alkyl, halogen, C₁-C₄-alkylsulfanyl, C₁-C₄-alkylsulfinyl and C₁-C₄-alkylsulfonyl, phenyl, C₁-C₆-alkyl substituted by a nonaromatic heterocyclic radical having 5 or 6 ring atoms and 1 or 2 heteroatoms independently of one another selected from N, O and S, C₁-C₄-alkylsulfanyl, C₁-C₄-alkylsulfinyl or C₁-C₄-alkylsulfonyl, or is phenyl which has one or two substituents independently of one another selected from C₁-C₄-alkyl, halogen, C₁-C₄-alkylsulfanyl, C₁-C₄-alkylsulfinyl and C₁-C₄-alkylsulfonyl,
R¹ and R² together are -CH₂CH₂- or -CH₂CH₂CH₂-,
R³ is halogen substituted phenyl,
R⁴ is 4-pyridyl which is substituted by amino, C₁-C₄-alkylamino, phenyl-C₁-C₄-alkylamino or R⁵CONR⁶-, where R⁵ is C₁-C₄-alkyl, phenyl which may have one or two substituents independently of one another selected from C₁-C₄-alkyl, C₁-C₄-alkoxy and halogen, or C₃-C₆-cycloalkyl and R⁶ is H, C₁-C₄-alkyl or benzyl, and
or an optical isomer or physiologically acceptable salt thereof.

9. A compound as claimed in claim 8, wherein R¹ is as defined in claim 5 or 6.

10. A compound as claimed in claim 8 or 9, wherein R² is as defined in claim 7.

11. A compound as claimed in any of claims 8 to 10, wherein R⁴ is as defined in claim 3.

12. A pharmaceutical composition, comprising at least one compound as claimed in any of claims 1 to 11, if appropriate together with one or more pharmaceutically acceptable carriers and/or additives.

13. The use of at least one compound as claimed in any of claims 1 to 11 for preparing a pharmaceutical composition for treating disorders associated with a disturbed immune system.

## Revendications

1. Composés d'imidazole substitués par 2-thio de formule I 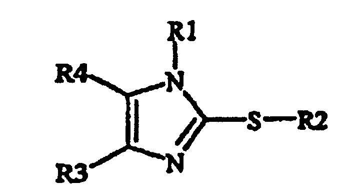 dans laquelle
R¹ est choisi parmi :
alkyle en C₁-C₆, le cas échéant substitué par un ou deux groupes hydroxy ou alcoxy en C₁-C₄, ou un radical hétérocyclique non aromatique avec 5 ou 6 atomes de cycle et 1 ou 2 hétéroatomes, choisis indépendamment les uns des autres, parmi N, O et S,
alcényle en C₂-C₆,
cycloalkyle en C₃-C₆,
aryle, le cas échéant substitué par un ou plusieurs atomes d'halogène ou un groupe alkylsulfanyle en C₁-C₄,
amino-C₁-C₄-alkyle, où le groupe amino est le cas échéant substitué par un ou deux groupes alkyle en C₁-C₄,
aminoaryle, où le groupe amino est le cas échéant substitué par un ou deux groupes alkyle en C₁-C₄,
aryl-C₁-C₄-alkyle ou
un radical hétérocyclique aromatique ou non aromatique ayant 5 ou 6 atomes de cycle et 1 ou 2 hétéroatomes, qui sont choisis indépendamment les uns des autres, parmi N, O et S, qui est substitué le cas échéant par 1, 2, 3 ou 4 groupes alkyle en C₁-C₄, un groupe aryle ou aryl-C₁-C₄-alkyle,
R² est choisi parmi :
alkyle en C₁-C₆,
phényl-C₁-C₄-alkyle, où le groupe phényle peut présenter un ou deux substituants, qui sont choisis indépendamment l'un de l'autre parmi les groupes alkyle en C₁-C₄, halogène, alkylsulfanyle en C₁-C₄, alkylsulfinyle en C₁-C₄ et alkylsulfonyle en C₁-C₄,
alcényle en C₂-C₆,
alcényle en C₂-C₆, qui est substitué par un ou deux atomes d'halogène et/ou groupes phényle, où le groupe phényle peut être, indépendamment, substitué par un ou deux groupes alkyle en C₁-C₄ ou atomes d'halogène,
alkinyle en C₂-C₆,
alkinyle en C₂-C₆, qui est substitué par un groupe phényle, qui peut être substitué le cas échéant par un ou deux groupes alkyle en C₁-C₄ ou atomes d'halogène,
alkyle en C₁-C₆, qui est substitué par un radical hétérocyclique non aromatique ayant 5 ou 6 atomes cycliques et 1 ou 2 hétéroatomes qui sont choisis indépendamment les uns des autres parmi N, O et S, alkylsulfanyle en C₁-C₄, alkylsulfinyle en C₁-C₄ ou alkylsulfonyle en C₁-C₄,
phényle ou
phényle, qui présente un ou deux substituants, qui sont choisis, indépendamment l'un de l'autre, parmi les groupes alkyle en C₁-C₄, halogène, alkylsulfanyle en C₁-C₄, alkylsulfinyle en C₁-C₄ et alkylsulfonyle en C₁-C₄,
R¹ et R², ensemble, représentent -CH₂CH₂- ou -CH₂CH₂CH₂-,
R³ représente un phényle substitué par un halogène,
R⁴ représente le 4-pyridyle, qui est substitué par un groupe amino, alkylamino en C₁-C₄, phényl-C₁-C₄-alkylamino ou R⁵CONR⁶, où R⁵ représente un alkyle en C₁-C₄, phényle, qui peut présenter un ou deux substituants, qui sont choisis, indépendamment l'un de l'autre, parmi les groupes alkyle en C₁-C₄, alcoxy en C₁-C₄ et halogène, ou représente un cycloalkyle en C₃-C₆ et R⁶ représente H, un alkyle en C₁-C₄ ou benzyle,
à la condition que, quand R¹ représente un aryl-C₁-C₅-alkyle ou amino-C₁-C₆-alkyle, où le groupe amino est le cas échéant substitué par un ou deux groupes alkyle en C₁-C₄, R² représente un alkylsulfonyl- ou alkylsulfinyl-aryl-C₁-C₅-alkyle,
et les isomères optiques et les sels physiologiquement acceptables de ceux-ci.

2. Composés selon la revendication 1, où le radical pyridyle est substitué en position 2.

3. Composés selon la revendication 1 ou 2, où R⁴ représente le 4-pyridyle qui est substitué par un groupe phényl-C₁-C₄-alkylamino.

4. Composés selon l'une des revendications précédentes, où R³ représente le 4-fluorophényle.

5. Composés selon l'une des revendications précédentes, où R¹ représente un alkyle en C₁-C₃, cycloalkyle en C₃-C₆ ou 2,2,6,6-tétraméthylpipéridinyle.

6. Composés selon la revendication 5, où R¹ représente un alkyle en C₁-C₃ ou 2,2,6,6-tétraméthylpipéridinyle.

7. Composés selon l'une des revendications précédentes, où R² représente un groupe alkyle en C₁-C₆ ou phényl-C₁-C₄-alkyle, où le groupe phényle est substitué par les groupes alkylsulfanyle en C₁-C₄, alkylsulfinyle en C₁-C₄ ou alkylsulfonyle en C₁-C₄.

8. Composés de formule I, dans laquelle R¹ représente un alkyle en C₁-C₆, le cas échéant substitué par un ou deux groupes hydroxy ou alcoxy en C₁-C₄, ou un radical hétérocyclique non aromatique ayant 5 ou 6 atomes de cycle et 1 ou 2 hétéroatomes, choisis indépendamment les uns des autres parmi N, O et S, représente un cycloalkyle en C₃-C₆, ou un radical hétérocyclique non aromatique ayant 5 ou 6 atomes de cycle et 1 ou 2 hétéroatomes, choisis indépendamment les uns des autres parmi N, O et S, le cas échéant substitué par 1, 2, 3 ou 4 groupes alkyle en C₁-C₄, un groupe aryle ou aryl-C₁-C₄-alkyle, R² représente un groupe alkyle en C₁-C₆ ou phényl-C₁-C₄-alkyle, où le groupe phényle peut présenter un ou deux substituants, choisis, indépendamment l'un de l'autre parmi les groupes alkyle en C₁-C₄, halogène, alkylsulfanyle en C₁-C₄, alkylsulfinyle en C₁-C₄ ou alkylsulfonyle en C₁-C₄, représente un groupe phényle, alkyle en C₁-C₆, qui est substitué par un radical hétérocyclique non aromatique ayant 5 ou 6 atomes cycliques et 1 ou 2 hétéroatomes, qui sont choisis indépendamment les uns des autres parmi N, O et S, alkylsulfanyle en C₁-C₄, alkylsulfinyle en C₁-C₄ ou alkylsulfonyle en C₁-C₄, ou représente un phényle, qui présente un ou deux substituants, qui sont choisis, indépendamment l'un de l'autre, parmi les groupes alkyle en C₁-C₄, halogène, alkylsulfanyle en C₁-C₄, alkylsulfinyle en C₁-C₄ et alkylsulfonyle en C₁-C₄,
R¹ et R², ensemble, représentent -CH₂CH₂- ou -CH₂CH₂CH₂-,
R³ représente un phényle substitué par un halogène,
R⁴ représente le 4-pyridyle, qui est substitué par un amino, alkylamino en C₁-C₄, phényl-C₁-C₄-alkylamino ou R⁵CONR⁶, où R⁵ représente un groupe alkyle en C₁-C₄, phényle, qui peut présenter un ou deux substituants, qui sont choisis, indépendamment l'un de l'autre parmi les groupes alkyle en C₁-C₄, alcoxy en C₁-C₄ et halogène, ou représente un cycloalkyle en C₃-C₆ et R⁶ représente H, alkyle en C₁-C₄ ou benzyle,
et les isomères optiques et les sels physiologiquement acceptables de ceux-ci.

9. Composés selon la revendication 8, où R¹ a les significations indiquées dans l'une des revendications 5 ou 6.

10. Composés selon la revendication 8 ou 9, où R² a les significations indiquées dans la revendication 7.

11. Composés selon l'une des revendications 8 à 10, où R⁴ a les significations indiquées dans la revendication 3.

12. Produits pharmaceutiques, contenant au moins un composé selon l'une des revendications 1 à 11, le cas échéant conjointement avec un ou plusieurs supports et/ou additifs pharmaceutiquement acceptables.

13. Utilisation d'au moins un des composés selon l'une des revendications 1 à 11, pour la préparation d'un produit pharmaceutique pour le traitement des maladies qui sont associées à un trouble du système immunitaire.
